# EUROPEAN PATENT SPECIFICATION

(11) **EP 1 456 241 B1**
(45) Date of publication and mention of the grant of the patent: **16.03.2005**
(21) Application number: 02795261.3
(22) Date of filing: 20.12.2002
(51) Int. Cl.: C07K 19/00

(54) **MONO-AND DUAL ANTI-CD4-RANTES CHEMOKINE/CYTOKINE CONSTRUCTS**
EINZELNE UND DUALE ANTI-CD4-RANTES CHEMOKIN/ZYTOKIN KONSTRUKTE
COMPOSES SIMPLES ET DOUBLES DE CHEMOKINE/CYTOKINE ANTI-CD4-RANTES

(30) Priority: 21.12.2001 EP 01130746
(43) Date of publication of application: 15.09.2004
(73) Proprietor: Micromet AG, 81477 München (DE)
(72) Inventor: MACK, Matthias, 93051 Regensburg (DE); SCHLOENDORFF, Detlef, 80803 München (DE)
(74) Representative: VOSSIUS & PARTNER
(86) International application number: PCT/EP2002/014683
(87) International publication number: WO 2003/054017

(56) References cited:
- WO-A-00/35409
- WO-A-00/53633
- WO-A-01/43779
- WO-A-02/20615
- BRUHL H ET AL: "Depletion of CCR5-expressing cells with bispecific antibodies and chemokine toxins: a new strategy in the treatment of chronic inflammatory diseases and HIV" JOURNAL OF IMMUNOLOGY, THE WILLIAMS AND WILKINS CO. BALTIMORE, US, vol. 166, no. 4, 15 February 2001 (2001-02-15), pages 2420-2426, XP002197003 ISSN: 0022-1767

## Description

The present invention relates to a chemokine construct comprising a scFv anti-CD4 and a RANTES chemokine or a functional fragment thereof, whereby said chemokine construct has the structural domain form of (a) RANTES-linker-(VH) anti-CD4-linker-(VL) anti-CD4; or (b) (VL) anti-CD4-linker-(VH) anti-CD4-linker-RANTES, or (c) (VH) anti-CD4-linker-(VL) anti-CD4-linker RANTES, whereby in the alternatives (b) and (c), the linker between (VH) anti-CD4 and RANTES or between (VL) anti-CD4 and RANTES does not terminally comprise a methionine, a lysine or an isoleucine directly adjacent to the first amino acid of RANTES. Furthermore, polynucleotides encoding these chemokine constructs as well as vectors comprising these polynucleotides are disclosed. The invention also relates to specific uses of these constructs and to pharmaceutical compositions.

Human immunodeficiency virus-type 1 (HIV-1), the most common cause of AIDS, has infected more than 50 million individuals (including those who have died), and the rate of new infections is estimated at nearly 6 million per year (AIDS Epidemic Update: December 1999 (UNAIDS, Geneva, 1999), www.unaids.org). Equally disturbing are the uncertainties of the epidemic to come. Although sub-Saharan Africa remains the global epicenter, rates of infection have increased in recent times in the former Soviet Union and parts of south and southeast Asia, including India and China, where literally hundreds of millions of individuals are potentially at risk. In the United States, new waves of infection have been recognized in women, minorities, and younger generations of gay men. Combination antiretroviral therapy has afforded many people clinical relief, but the costs and toxicities of treatment are substantial, and HIV-1 infection remains a fatal disease. Moreover, the vast majority of infected people worldwide do not have access to these agents. Thus, although the demographics (and, in some instances, the natural history) of AIDS have changed, the epidemic is far from over; instead, it is evolving, expanding, and posing ever greater challenges.

Human immunodeficiency virus (HIV) cannot enter human cells unless it first binds to two key molecules on the cell surface, CD4 and a co-receptor. The co-receptor that is initially recognized is CCR5, later in the life cycle of the virus another chemokine receptor CXCR4 becomes the co-receptor for HIV-1 (D'Souza & Harden, Nature Med. 2, 1293-1300 (1996); Premack & Schall, Nature Med. 2, 1174-1178; Fauci, Nature 384, 529-534 (1996)). The HIV-1 strains that cause most transmissions of viruses by sexual contact are called M-tropic viruses. These HIV-1 strains (also known as NSI primary viruses) can replicate in primary CD4+ T-cells and macrophages and use the chemokine receptor CCR5 (and, less often, CCR3) as their coreceptor. The T-tropic viruses (sometimes called SI primary) can also replicate in primary CD4+ T-cells but can in addition infect established CD4+ T-cell lines in vitro, which they do via the chemokine receptor CXCR4 (fusin). Many of these T-tropic strains can use CCR5 in addition to CXCR4, and some can enter macrophages via CCR5. at least under certain in vitro conditions (D'Souza & Harden, Nature Med. 2, 1293-1300 (1996); Premack & Schall, Nature Med. 2, 1174-1178; Fauci, Nature 384, 529-534 (1996)). Whether other coreceptors contribute to HIV-1 pathogenesis is unresolved, but the existence of another coreceptor for some T-tropic strains can be inferred from in vitro studies. Because M-tropic HIV-1 strains are implicated in about 90% of sexual transmissions of HIV, CCR5 is the predominant coreceptor for the virus in patients; transmission (or systemic establishment) of CXCR4-using (T-tropic) strains is rare (D'Souza & Harden, Nature Med. 2, 1293-1300 (1996); Premack & Schall, Nature Med. 2, 1174-1178; Fauci, Nature 384, 529 - 534 (1996), Paxton et al. , Nature Med. 2, 412-417 (1996); Liu et al., Cell 86, 367-377 (1996); Samson et al., Nature 382, 722-725 (1996); Dean et al., Science 273, 1856 - 1862 (1996); Huang et al., Nature Med. 2, 1240-1243 (1996)). However, once SI viruses evolve in vivo (or if they are transmitted), they are especially virulent and cause faster disease progression (D'Souza & Harden, Nature Med. 2, 1293-1300 (1996); Premack & Schall, Nature Med. 2, 1174-1178; Fauci, Nature 384, 529-534 (1996), Schuitemaker et al., J. Virol. 66, 1354-1360 (1992); Connor et al., J. Virol. 67, 1772-1777 (1993); Richman & Bozzette, J. Infect. Dis. 169, 968-974 (1994); Connor et al., J. Exp. Med. 185, 621-628 (1997); Trkola et al., Nature 384, 184 -187 (1996)).

In summary, human immunodeficiency virus type 1 (HIV-1) uses a coreceptor together with CD4 to enter CD4+ target cells. The chemokine receptors CXCR4 and CCR5 have been found to be the major coreceptors for T-cell line-tropic and macrophage-tropic HIV-1 strains, respectively. The cellular receptors are CCR5 for RANTES and CXCR4 for SDF-1. CCR5 is expressed on CD4+ memory cells and CD8+ T cells. CCR5 is also expressed on activated monocytes and macrophages. The mechanism of virus entry is based on the interaction of the HIV-1 envelope glycoprotein gp120 with CD4 and a coreceptor, leading to a membrane fusion reaction between the viral envelope and the plasma membrane of the target cell. Blocking of the binding of gp120 to CD4 and a coreceptor represents an appropriate strategy to prevent HIV-1 infection of CD4+ T-cells.

The chemokine receptor CCR5 has an important function in chemotaxis of lymphocytes, monocytes, and dendritic cells. Binding of RANTES to CCR5 can induce two different kinds of signalling pathways. Signalling through CCR5 induces transient calcium influx, cell polarization and chemotaxis and thereby a proinflammatory response. Increased RANTES expression is associated with inflammatory disorders. Associated with its proinflammatory activity is a low local tolerance as shown by a strong inflammatory response after intradermal injection (estimated local concentration of ~1 µM) of hRANTES in dog (Meurer et al., 1993, J Exp Med *178*, 1913-1921).

CCR5 is also a major coreceptor in most macrophage-tropic HIV-1 infections that is primarily used in the early phase of infection. Binding of RANTES to CCR5 mediates suppression of HIV infection, however CCR5 signalling does not seem to be required for this suppressive effect (Alkhatib et al, 1997, Virology 234, 340-348; Bacon et al., 1995, Science 269, 1727-1730; Oravecz et al., 1996, J Immunol 157, 1329-1332). The HIV suppressive effect of RANTES might instead result from blocking HIV gp120 binding sites or downregulation of CCR5 at the cell surface.

As an alternative to CCR5 (Deng et al., 1996, Nature 381, 661-666; Dragic et al., 1996, Nature *381*, 667-673) HIV-1 (T-tropic strains; X4 strains; SI: syncytium-inducing) can also use CXCR4 (fusin) as coreceptor (Feng et al., 1996, Science 272, 872-877). Similar to RANTES/CCR5, HIV-1 infection through CXCR4 can be blocked by SDF-1 (Bleul et al., 1996, Nature 382, 829-833; Obenin et al, 1996, Nature 382,833-835).

During clinical progression, viruses shifted from non-syncytia inducing to syncytia inducing phenotype and in parallel loose sensitivity to beta-chemokines (Jansson et al., 1996, Proc Natl Acad Sci U S A *93*, 15382-15387). It has been shown that blockade of one coreceptor can induce a shift in receptor specificity of virus population (Este et al, 1999, J Virol 73, 5577-5585). Since X4 strains are more pathogenic than R5 strains, blocking of CCR5 alone might therefore lead to a shift in receptor specificity and thus to more severe pathology. Indeed, combination of AOP-RANTES (CCR5 antagonist) with Met-SDF-1 (CXCR4 antagonist) inhibits mixed infections with R5 and X4 viruses to >95%, whereas single chemokines only inhibited to 32-61% (Rusconi et al, 2000, J Virol 74, 9328-9332).

The chemokine receptors CXCR4 and CCR5 are used as co-receptors by the T cell-tropic and macrophage-tropic HIV-1 strains, respectively, for entering their host cells. Viral entry can be inhibited by the natural ligands for CXCR4, the CXC chemokine SDF-1 and for CCR5, the CC chemokines RANTES, MIP-1alpha MIP-1beta, LD78beta and MCP-2. Small molecular CXCR4 antagonists have been developed. The bicyclam derivate AMD 3100 has proved to be a highly specific CXCR4 antagonist, which consistently blocks the outgrowth of all T cell tropic and dual-tropic HIV variants that use CXCR4 for entering the cells (Donzella et al., 1998, Nat Med 4, 72-77; Este et al, 1999, J Virol 73, 5577-5585; Schols et al, 1997, Antiviral Res 35, 147-156; Schols et al, 1997, J Exp Med 186, 1383-1388). AMD3100 was selected as the clinical drug candidate, which, after initial Phase I (safety) studies, has proceeded to Phase II (efficacy) trials. The first non-peptidic compound that interacts with CCR5, and not with CXCR4, is a quaternary ammonium derivative, called TAK-779, which also has potent but variable anti-HIV activity (Bab et al. ,1999, Proc Natl Acad Sci U S A 96, 5698-5703). In addition, SCH-C is an orally available small molecular CCR5 antagonist and a potent inhibitor of HIV-1 infection (Strizki et al. 2001, Proc Natl Acad Sci U S A. 98:12718-12723). HIV entry/fusion inhibitors are promising new antiviral agents to combat AIDS. However for optimum therapeutic effects they will need to be used in combinations with other antivirals targeting the HIV replication cycle, such as reverse transcriptase and protease (summarized in De Clercq & Schols, 2001, Antivir. Chem. Chemother;12 Suppl 1, 19-31).

Naturally occurring antibodies directed against the CCR5 coreceptor for HIV-1 which were isolated from normal human IgG were shown to inhibit the binding of RANTES to macrophages (Bouhlal et al., 2001, J. Immunol., 166, 7606-7611). Affinity-purified anti-CCR5 Ig further inhibited infection of lymphocytes and monocytes/macrophages with primary and laboratory-adapted strains of HtV-1, but did not inhibit infection with X4-tropic HIV. Binding of HIV gp120 to CD4 can be blocked by antiCD4 antibodies (Leu3a and OKT4A), however immunosuppression through depletion of CD4+ cells results as a side effect. US5871732 (priority date: 1991-11-27, issued US: 1999-2-16) claims an antiCD4 antibody (5A8) without immunosuppressive side effects.

MT413 was described to be efficient in inhibiting cellular infection with HIV-1 even after viral attachment to the cell membrane (Davis et al., 1992, Nature *358*, 76-79; Rieber et al., 1992, Proc Natl Acad Sci U S A 89, 10792-10796; Rieber et al., 1990, Lancet 336, 1007-1008) and its sequence was released (Weissenhom et al., 1996, Hybridoma 15, 117-124) (GenBank X65093 & X65086; deposited 1997-2-26).

In addition to signaling through CCR5 and other chemokine receptors, RANTES can also act as a powerful antigen-independent activator of T-cells by signaling through TCR-components and protein tyrosine kinases leading to a sustained calcium influx (Bacon et al., 1995, Science 269, 1727-1730; Dairaghi et al., 1998, J Immunol *160*, 426-433). This type of signalling only occurs at high concentrations of RANTES (>100 nM-1 µM). At such high concentrations, RANTES can actually enhance HIV infection. Both activities are critically dependent on RANTES tendency to self-aggregate into larger, ill-defined oligomers. However, experimental conditions were described, where RANTES even at higher concentrations forms a dimer (Chung et al., 1995, Biochemistry, 34:9307-14; Wilken et al., 1999, Chem Biol., 6:43-51). Rantes with Glu26 or Glu66 mutated has a diminished tendency to aggregate (< tetramer, dodecamers), does not activate leukocytes or enhance HIV infection but retains its receptor binding activity and CCR-mediated biological activity (Appay et al., 1999, J Biol Chem 274, 27505-27512; Czaplewski et al., 1999, J Biol Chem 274, 16077-16084; Graham et al., 1994, J Biol Chem 269, 4974-4978).

For treatment of HIV-1 infection a RANTES antagonists with diminished CCR5 signalling activity (and thereby reduced proinflammatory activity) but intact HIV-suppressive activity and CCR5 binding affinity would be preferred. Several antagonists have been described resulting from chemical modifications, elongations, deletions or mutations of RANTES's N-terminus.

AOP-RANTES has increased antiviral effect through enhanced receptor internalization but maintains signalling function (Mack et al., 1998, J Exp Med 187, 1215-1224; Oppermann et al., 1999, J Biol Chem 274, 8875-8885; Simmons et al., 1997, Science 276, 276-27). US6168784, andWO9911666 claim different kinds of RANTES, including NNY-RANTES (Mosier et al., 1999, J Virol 73, 3544-3550). Met-RANTES is a complete RANTES antagonist and is less effective than RANTES on HIV suppression (Simmons et al. 1997, Science 276, (5310), 276-279). Leu-RANTES has a 2-3 fold higher antiviral effect and binds more stably to CCR5 than RANTES wildtype. This molecule is a very weak agonist, but a potent antagonist (Polo et al., 2000, Eur J Immunol 30, 3190-3198). C1C5-RANTES has about 5 fold higher antiviral effect but other mutants have much lower antiviral effect (C1-RANTES, A3-RANTES, R6-RANTES). C1C5-RANTES is also a weak agonist and a potent antagonist (Polo et al., 2000, Eur J Immunol 30, 3190-3198). RANTES (9-68) has low chemotactic and weaker antiviral activity than RANTES wildtype. This molecule simply seems to be a weaker binder to CCR chemokine receptors (Gong et al., 1996, J Biol Chem 271, 10521-10527; Polo et al., 2000, Eur J Immunol 30, 3190-3198). Challita-Eid et al. (1998, AIDS Res Hum Retroviruses 14, 1617-1624) also constructed a RANTES-IgG fusion protein with the aim of inhibiting HIV infection.

Furthermore Brühl et al. described a RANTES-toxin (PE) fusion protein for treatment of inflammatory diseases and HIV (Brühl et al., 2001, J Immunol *166*, 2420-2426).

Recently, a chimeric molecule consisting of the eight amino terminal residues of MIP-1alpha preceding the CC motif followed by the remaining sequence of RANTES was described (Blanpain et al., 2001, Leukoc Biol, 977-85) which was able to induce chemotaxis of human monocytes. MIP/RANTES did not bind to CCR3 but had equivalent affinity for CCR5 binding with an EC(50) of 1.12 nM. MIP/RANTES induced endocytosis of CCR5 in PBMC in a manner equipotent to RANTES and was able to inhibit CCR5 using HIV-1 strains. These data suggest that the N-terminus of RANTES is not involved in CCR5 binding, but it is essential for CCR1 and CCR3.

Trafficking of inflammatory T cells into the central nervous system (CNS) plays an important role in the pathogenesis of multiple sclerosis. The directional migratory ability of peripheral T cells is associated with interactions of chemokines with their receptors expressed on T cells. It was shown that aberrant migration of multiple sclerosis-derived T cells toward RANTES and MIP-1 alpha resulted from overexpression of their receptors (CCR5) and could be blocked by anti-CCR5 antibodies (Zang et al. , 2000, Brain 123, 1874-82).

Recently, a role of RANTES in the cartilage degradation was described. Normal chondrocytes produce RANTES and express RANTES receptors. RANTES and CCR5 were markedly increased in osteoarthritic cartilage and after in vitro treatment of normal chondrocytes with IL-1. Chondrocyte activation and cartilage degradation were identified as novel biologic and pathogenetic activities of this chemokine (Alaaeddine et al., 2001, Arthritis Rheum , 44, 1633-43).

Recent studies suggest that chemokines are involved in the formation of nonneoplastic leukocytic infiltrates in Hodgkin Disease (HD) (Buri et al.., 2001, Blood, 97, 1543-8).The expression of RANTES, MCP-1, MIP-1alpha and MIP-1beta was markedly enhanced, but RANTES was expressed almost exclusively by T cells whereas the expression of MCP-1, MIP-1alpha, and MIP-1beta was confined largely to macrophages. Compared to controls CCR3 and CCR5 were highly expressed in T cells. In HD lymph nodes, CCR3 and CCR5 were also expressed in B cells, which normally do not express these receptors.

Viral spreading in HIV-1 infection can be prevented by blocking entry of HIV-1 via cellular receptors like CD4 and CCR5. There are different approaches using chemokine receptor ligands or antibodies directed against these receptor molecules. RANTES as a ligand of CCR5 and anti-CD4 monoclonal antibodies proved to be effective in blocking entry of HIV-1 in CD4+ T cells but both compounds showed severe side effects. CD4 specific antibodies can induce depletion of the CD4+ T cells (Rep et al., J Clin Invest., 1997, 99, 2225-2231; van der Lubbe et al., J. Autoimmun., 1997, 10, 87-97; Knox et al., Blood, 1996, 87, 893-899; Moreland et al., Arthritis Rheum., 1995, 38, 1581-8; Moreland et al., Arthritis Rheum., 1994, 37, 834-838) and do not differentiate between subpopulations of CD4+ T cells. The use of RANTES or modifications thereof is described to initiate inflammatory processes (reviewed in Ward & Westwick, 1998, Biochem J 333, 457-470; Appay & Rowland-Jones, 2001, Trends Immunol 22, 83-87). Application of RANTES in vivo may also paralyze the function of CCR5 and thereby induce an immune-suppression. As a consequence the subpopulations of memory CD4+ T cells and cytotoxic CD8+ cells which are also positive for CCR5 are reduced leading to a diminished cellular immune response. This detrimental effect on the T cell activity is a hallmark of therapeutic approaches using RANTES and anti-CD4 monoclonal antibodies for blocking HIV-1 entry.

Using antibodies or antibody-fragments against CCR5 in combination with antibodies against CD4, as inter alia described in WO 01/43779, has the major disadvantage that antibodies or antibody fragments against CCR5 are only weak inhibitors of HIV-infection compared to the chemokines RANTES, MIP-1alpha or MIP-1beta (own data). In addition, CCR5 antibodies are not able to internalize CCR5 from the surface of PBMC, while the chemokines RANTES, MIP-1alpha or MIP-1beta very efficiently induce internalization of CCR5. Novel CCR5 antibodies were tested for their ability to internalize CCR5 from the surface of PBMC and found that only one CCR5 antibody (MC-1, as documented in the appended examples) showed a very weak internalization of CCR5 on PBMC. In addition ,this CCR5 antibody MC1 could internalize CCR5 only as a bivalent (intact) antibody and did not internalize CCR5 as F(ab) fragment or scFv-fragment. Internalization of CCR5 has the advantage that CCR5 disappears from the cell surface and is no longer available as co-receptor for HIV-1. In contrast mere blockade of CCR5, e.g. by CCR5 antibodies, depends on a steric hindrance to prevent binding of HIV-1 to CCR5. The steric hindrance is only effective against some M-tropic HIV-1 strains while internalization of CCR5 can block HIV-infection with all HIV-1 strains that use CCR5 (Mack et al. J. Exp. Med. 1998; 187:1215-24).

Similarly, there are patients suffering from inflammatory or autoimmune disease that do not respond to currently available therapies. In other cases, the conventional therapy has to be stopped due to intolerable side effects.

In many diseases like arthritis, inflammatory and autoimmune diseases, e.g. inflammatory renal diseases, inflammatory bowel diseases, multiple sclerosis and transplant rejection, current treatment methods have many limitations. For example, agents used in inflammatory and autoimmune diseases include anti-inflammatory and general immunosuppressive agents like azathioprine, cyclophosphamide, glucocorticoids like prednisone and corticosteroids; immunosuppressants like cyclosporin A, Tacrolimus (FK506), Sirolimus (Rapamycin); and protein drugs like calcineurin, beta-interferon, anti-TNF alpha monoclonal antibodies (remicade). These agents show general immunomodulating effects and therefore efficacy and side effects profiles can pose severe limitations for the treatment options (Harrison's Principles of Internal Medicine, eds. Fauci et al., 14^{th} edition, McGraw-Hill publisher).

Therefore, the technical problem underlying the present invention was to provide for improved means and methods for the medical intervention in inflammatory diseases, autoimmune disorders and/or viral infections of immune cells, e.g. HIV infections.

The solution to this technical problem is provided by the embodiments characterized in the claims.

Accordingly, the present invention relates to a chemokine construct comprising a scFv anti-CD4 and a RANTES chemokine (or a functional fragment thereof), whereby said chemokine construct has the structural domain form of (a) RANTES-linker-(VH) anti-CD4-linker-(VL) anti-CD4; or (b) (VL) anti-CD4-linker-(VH) anti-CD4-linker-RANTES, or (c) (VH) anti-CD4-linker-(VL) anti-CD4 -linker-RANTES, whereby in the alternatives (b) and (c), the linker between (VH) anti-CD4 and RANTES or between (VL) anti-CD4 and RANTES does not terminally comprise a methionine, a lysine or an isoleucine directly adjacent to the first amino acid of RANTES.

The term "RANTES" as used herein above relates to functional RANTES, whereby said function comprises the binding to CCR5. Therefore, the term also comprises fragments of RANTES and/or derivatives of RANTES which fulfill the above described requirements and are capable of binding CCR5. RANTES as well as RANTES fragments and RANTES derivatives are known in the art and are illustrated herein below as well as in the appended examples. Functional fragments of RANTES are defined herein below and comprise, inter alia RANTES fragments "delta2" or "delta3", which miss 2, respectively 3, amino acids in its N-terminal part. As discussed above, the term RANTES and RANTES constructs as employed herein does not comprise RANTES modifications of the form "Met-RANTES'' or ''AOP-RANTES".

The term "scFv anti-CD4" relates to a single chain construct as further defined herein below, comprising at least a (VL) and a (VH). The generation of such single chain constructs is known in the art and furthermore illustrated in the appended examples. The scFv parts as employed herein are directed against and/or interact with the CD4 antigen, and comprises VL and VH domains of an antibody specific to said CD4 antigen and/or epitopes derived therefrom, whereby said VH and VL relates to the variable regions of the heavy and light chain of said antibody, respectively. Useful anti-CD4 antibodies in accordance with this invention are, in particular but not limiting, antibodies directed against CD4 which are capable of competing with the Viral gp120 molecule for interaction/binding with CD4, an adhesion molecule that binds to class II molecules.

The term "structural domain form" as used herein and described, relates to a construct comprising different structural and functional parts, like the RANTES part, the scFv anti-CD4 part or the linker part described herein and is meant to show the structural form of the constructs of this invention. Thereby, the structural form laid down herein relates to constructs wherein the first part is the N-terminal part and the last part is the C-terminal part. Accordingly, in the constructs (a), (b) and (c) described herein above, the structural form of the inventive construct (a) is RANTES at the N-terminus and the (VL) of the anti-CD4 at the C-terminus. For the inventive construct (b) and (c), RANTES is C-terminally located and (VL) of anti-CD4 or (VH) of anti-CD4, respectively, are on the N-terminus. As will be explained in detail herein below, neither RANTES nor the (VH) or (VL) have to be the extreme N-or C-terminal part of the construct of the invention. It is, e.g., envisaged that further parts are linked at the N-or C-terminus. These additional parts, may, inter alia, be tags or further functional domains, like further cytokines/chemokines.

The term "linker" as employed in accordance with this invention comprises in particular peptide linkers. As exemplified in the appended examples, particularly useful "linkers/linker domains" in accordance with this invention are stretches of glycine and serine residues, for example Gly-Gly-Gly-Gly-Ser (SEQ ID NO:51) and multimers thereof, like, inter alia, Gly₄SerGly₄SerGly₄ (SEQ ID NO:52) or Gly₄SerGly₄Ser (SEQ ID NO:53). As will be detailed herein below, it is preferred that the linker between RANTES (or its functional fragment) and the scFv anti-CD4 is Gly₄SerGly₄Ser (SEQ ID NO:53), and may comprise, at the nucleic acid level of said construct a BspE1 and BamH1 restriction site. The linker region between the (VH) and (VL) parts of the scFv domain of the construct may comprise, Gly₄SerGly₄SerGly₄ (SEQ ID NO: 52).

As described herein above, in the chemokine constructs as shown in the alternatives (b) and (c), the linker between (VH) anti-CD4 and RANTES or between (VL) anti-CD4 and RANTES does not terminally comprise a methionine (Met), a lysine (Lys) or an isoleucine (Ile) directly adjacent to the first amino acid of RANTES. Furthermore, it is preferred that said last amino acid of the linker directly adjacent to RANTES or its functional fragment does neither comprise a basic amino acid, like arginine (Arg) or histidine (His) nor a large amino acid, like the aromatic amino acids phenylalanine (Phe), tryptophan (Trp) or tyrosine (Tyr). In addition, a less preferred amino acid at this position is proline (Pro). In contrast, preferred amino acids at the last position of said linker directly adjacent to the RANTES or its functional fragment are glycine (Gly), glutamic acid (Glu), glutamine (Gln) or leucine (Leu).

The term "terminally" as employed herein above means that the last C-terminal amino acid of said linker is directly followed by an N-terminal amino acid of a given RANTES sequence. Preferably, said term relates to the fact that said "terminal" amino acid sequence of the linker is directly adjacent to the first amino acid sequence of the RANTES sequence. Most preferably, but not limiting, said "terminal" amino acids of the linker is directly linked to RANTES and no further amino acid(s), not belonging to the amino acid sequence of said linker or said RANTES sequence, are comprised between the linker and RANTES. The feature "terminally" as employed herein also means that the last amino acid of the C-terminus of the linker, which is directly adjacent to the RANTES sequence is not a methionine, a lysine or an isoleucine.

Yet, it is also envisaged in accordance with this invention that the inventive chemokine construct comprises a scFV anti-CD4 and a RANTES chemokine (or a functional fragment thereof), whereby said chemokine construct has the structural domain form of (a) RANTES-linker-(VH) anti-CD4-linker-(VL) anti-CD4; or (b) (VL) anti-CD4-linker-(VH) anti-CD4-linker-RANTES, or (c) (VH) anti-CD4-linker-(VL) anti-CD4 -linker-RANTES, whereby in the alternatives (b) and (c), the linker between (VH) anti-CD4 and RANTES or between (VL) anti-CD4 and RANTES does not comprise a methionine, a lysine or an isoleucine directly adjacent to the first amino acid of **RANTES.**

In accordance with this invention it was surprisingly found that specific chemokine constructs as defined lead to a specific and efficient internalization of CCR5 without the undesired effect that a reduction of T-cell activity occurs. This surprising finding now enables the person skilled in the art to provide for specific treatment and/or prevention methods for disease and disorders, wherein CCR5 is involved, expressed or even over-expressed. Such diseases comprise HIV-infections/AIDS, as well as inflammatory and autoimmune diseases, like multiple sclerosis, rheumatoid arthritis, allergic reactions, inflammatory renal disease, inflammatory bowel disease, skin diseases or transplant rejections. As detailed herein below, the constructs of the invention are useful when a minor immunosuppressive effect is desired. Accordingly, in disorders like autoimmune diseases, transplant rejections, and inflammations, therapeutic approaches with the constructs of the invention are particularly envisaged in co-therapeutic settings with further medicaments.

As detailed herein and illustrated in the appended examples, it was surprisingly found that not all RANTES-anti-CD4 constructs are capable of elucidating an internalization of CCR5. A chemokine construct comprising the structural domain form RANTES-linker-(VL) anti-CD4-linker-(VH) anti-CD4 is, e.g. not capable of binding to CD4 and/or CCR5 and/or of CCR5 internalization. As discussed herein, particularly preferred chemokine constructs are comprising the structural domain form RANTES-linker-(VH) anti-CD4-linker-(VL) anti-CD4; or (b) (VL) anti-CD4-linker-(VH) anti-CD4 - linker-RANTES, or (c) (VH) anti-CD4-linker-(VL) anti-CD4-linker-RANTES, whereby the construct of attemative (a) is also referred to as "RM" and the construct of alternative (b) is also referred to as "MR". The appended examples document that it was, inter alia, found that the constructs of the invention, like RM, are specific for CD4⁺T cells and much more effective than RANTES alone when CCR5 internalization is evaluated. This was, inter alia, documented when internalization assays for CCR5 were performed which comprise preincubating PBMCs with RANTES or constructs of the invention on ice followed by wash-out of RANTES or the inventive constructs and further incubation of the cells at 37°C to induce internalization; see appended examples and figures. This experiment imitates the situation in vivo: unbound constructs were removed according to their plasma half lives and only the constructs that are bound to cells are still present. The inventive constructs are present for prolonged periods of time on CD4+ T cells and are capable of inducing internalization of CCR5 molecules that come into proximity of the inventive constructs bound to CD4. Therefore, the inventive constructs preferentially induce internalization of CCR5 molecules that are in close proximity to CD4 molecules, while CCR5 molecules that are not in the proximity of CD4 are not affected. As, inter alia, HIV-1 also depends on the close proximity of CD4 and CCR5 for infection, the simultaneous inhibition of CD4 and CCR5 by the inventive constructs selectively interfere with the HIV-1 receptors. Indeed, the constructs of the invention comprising RANTES and anti-CD4 were shown to be significantly more efficient in HIV infection blocking than the combination of RANTES and CD4scFv. Accordingly, major advantages of the present invention are that the memory T cell subset remains intact, no unselective blocking of all CCR5+ T cells occurs, internalization of CCR5 is preferentially induced in CD4+ cells expressing CCR5 in close proximity to the CD4 molecule and cytotoxic activity of CD8+ T cells is not affected.

RANTES-antiCD4 constructs of the invention specifically bind to CCR5 and CD4 receptors on T cells leading to an internalization of CCR5 from the cell surface. Internalization of CCR5 on CD4⁺ T cells was much more potent using the inventive constructs compared to RANTES alone. For example, the inventive constructs induce internalization of CCR5 molecules that are close to CD4 molecules, while CCR5 molecules that are not in the proximity of CD4 are less affected. Due to this capacity cytotoxic activity is not affected. This is, inter alia documented in the appended examples. It is shown that mixed lymphocyte reaction (MLR) is blocked by monoclonal anti-CD4 antibody MT413 but not by the inventive constructs, like "RM" or "MR". The CCR5 internalization induced by these constructs leads to a block of infection of CCR5+/CD4+ T cells with macrophage tropic HIV-1 strains but does not induce apoptosis of these T cells. The inventive constructs interact, as discussed herein above, simultaneously with CD4 and CCR5 and are capable of inducing internalization of CCR5 from the surface of CD4+ T cells, yet, only a weak internalization of CCR5 occurs in cells that express no CD4 (e.g. CD8+ T cells). Thereby CCR5 surface expression is reduced preferentially in the CD4+ CCR5+ cell population that is also the target of M-tropic HIV-1 strains. Accordingly, by employing the inventive constructs, the immunosuppressive effects of a widespread and unselective removal of CCR5 from the surface of all CCR5+ cells is avoided. In addition the inventive constructs contain no immunoglobulin Fc-portion, which is thought to be responsible for the depletion of CD4+ T cells in vivo after application of CD4 mAbs.

As far as the efficacy of the inventive constructs in treating or co-treating an HIV-infection/ AI DS or in treating or co-treating an immunological disease and disorder as described herein is concerned it was surprisingly observed that antibodies against CCR5 are not able to induce significant CCR5 internalization on PBMC. However, the inventive constructs, comprising an antibody fragment against CD4 and the chemokine RANTES are highly efficient in CCR5 internalization.

Surprisingly the inventive constructs have a higher efficacy in internalizing CCR5 on CD4+ T cells compared to RANTES alone, a prolonged efficacy compared to the short half-life of RANTES, a higher selectivity for CCR5 on CD4+ T cells since they interact with both molecules CCR5 and CD4 and, inter alia, a stronger blocking effect on HIV-1 infection compared to RANTES alone. Considering, for example the potency of inhibiting HIV infection (BAL isolate) by evaluating the relative reverse transcriptase activity (RT) with various molecules (20 nM), the following results were obtained: inventive construct RANTES-antiCD4 (RM):0.012; inventive construct antiCD4-RANTES (MR): <0.0076. Yet, RANTES alone showed in this assay a relative RT activity of 0.05 and an antiCD4 mab (MT413) alone of 0.46. Similarly, a tested anti-CD4 scFv construct leads to an RT activity of 0.55. Hereby it is of note that higher RT values indicate a higher production of HIV-1. With the SF162 isolate, the following relative reverse transcriptase activity values were measured with various molecules at 20nM: inventive RANTES-antiCD4 (RM): 0.013; inventive construct antiCD4-RANTES (MR): 0.010; RANTES alone: 0.31, MT413 mAb: 0.41 and an anti-CD4-scFv: 0.85.

Further envisaged advantages of the chemokine constructs of the present invention are that no resistance to HIV-1 strains as known for treatment with RT (reverse transcriptase) inhibitors is generated, since the inventive constructs block CD4 that is an essential receptor for all HIV-1 strains, and that the inventive constructs are easily and readily to be prepared compared to chemically linked antibodies.

As discussed herein above, not all RANTES-anti-CD4 constructs may provide for the advantagous features of the inventive constructs. Chemokine constructs of the present invention which are functional may readily be tested by assays as described herein above or in the appended examples. Further tests comprise, inter alia, binding tests, which may be carried out employing methods known in the art. For example, when evaluating the binding of the inventive constructs to CD4, the following not limiting methods may be employed. FACS analysis may be carried out: PBMC are incubated with the inventive constructs in different concentrations. After (a) washing step(s), the binding of the inventive construct may be detected with an antibody against a preferably introduced tag or against RANTES, followed by a secondary labeled antibody. ELISA analysis is envisaged: soluble CD4 may be used as antigenic target which is directly coupled to a solid matrix or support or via a capture antibody against CD4. After incubation with an inventive construct to be tested. detection could be performed with a secondary labeled antibody against RANTES or the potentially introduced tag. RIA (radioimmunoassay) assays with: radioactive labeled soluble CD4 may be used for detection of binding activity of the inventive constructs. Competition assay may be employed: PBMC are incubated with the inventive constructs in different concentrations. Without washing cells are incubated with labeled soluble CD4 (p.e. FITC). The lower the signal obtained with labeled CD4, the higher the amount of RM and MR bound to CD4+ cells. This assay may also be performed by ELISA, FACS or similar tests known in the art. Yet, the test to be employed for assaying the functionality of inventive constructs may also comprise the determination of binding of the inventive constructs to CCR5. Such tests comprise, but are not limited to internalization assays described in detail below and in the appended examples, ligand competition assays, calcium flux assays, migration assays, cAMP assays, GTP-binding assays, actin polymerization assays or transcription factor activation assays. The assays are known to the skilled artisan and are, inter alia, carried out as documented herein below.

An internalization assay for CCR5 may be carried out by incubating CCR5 expressing cells with the inventive constructs in different concentrations and the cell surface expression of CCR5 is analyzed by FACS using a labeled antibody against CCR5. Similarly, a ligand competition assay may comprise the incubation of CCR5 expressing cells (preferably on ice) with the inventive constructs and radioactive labeled RANTES. Cells are then washed and bound radioactivity is counted. A low detected radioactive signal is, accordingly, diagnostic for a high binding of the inventive construct. Calcium flux assay comprises the binding of inventive constructs to CCR5 expressing cells and the measurement of the induced calcium flux by methods known in the art. In migration assay the inventive constructs are tested for their ability to induce migration of CCR5 expressing cells. This assay may be performed in a Boyden-chamber assay or in the Transwell system. Furthermore, in cAMP assays, the increased concentration of intracellular cAMP after successful binding of the inventive constructs may be measured. The above mentioned GTP binding assays comprise the measurement of GTP bound to CCR5 since it is envisaged that binding of the inventive constructs leads to an increased binding of GTP. Actin polymerization assay may comprise the measurement of the polymerization of actin by in vitro methods known in the art. Binding of RANTES to CCR5 is known to induce the polymerization of actin molecules,which could be, inter alia, detected by antibodies specific for the polymerized form of actin. The successful binding of an inventive construct is envisaged to lead to a similar actin-polymerization effect. As mentioned herein above, a transcription factor activation assay may also be employed in order to evaluate the inventive constructs. The inventive constructs may induce increased expression of MAPkinase, leading to the activation of transcription factors. This increase may be measured by ELISA on protein level or by array technology on mRNA level.

Furthermore, the constructs of the invention can be tested, e.g. for blocking of HIV-infection, using in vivo experiments preferably in mammals, e.g. in mice or in chimpanzees. Different tissues or body fluids can be used e.g. blood, serum, spleen, lymph node or cerebrospinal fluid. One example of an in vivo assay which can be adapted for the present invention is described in Shultz et al. (2000) J Immunol. 164:2496-507).

Besides the above discussed assay systems, the inventive constructs may be evaluated for their binding abilities and/or their capability to induce CCR5 intemalization by further methods described in the art or by methods illustrated and exemplified in the appended examples.

In a preferred embodiment, the present invention relates to a chemokine as defined herein above, wherein the scFv anti-CD4 is derived from a monoclonal anti-CD4 antibody. Monoclonal antibodies directed against CD4 are known in the art and, inter alia, described in Weissenhom et al. (1996), Hybridoma 15, 117-124, reviewed in Riethmüller et al. (1992) Immunol. Rev. 129, 81-104.

In a most preferred embodiment such a monoclonal antibody is the antibody MT413, as described by Weissenhom et al. (1996) Hybridoma 15, 117-124. The corresponding sequences of this antibody are disclosed under GenBank accession numbers X65093 and X65086. MT413 is known to be efficient in inhibiting cellular infection with HIV-1 even after viral attachment to the cell membrane (Davis et al., 1992, Nature *358*, 76-79; Rieber et al., 1992, Proc Natl Acad Sci U S A *89*, 10792-10796; Rieber et al., 1990, Lancet 336, 1007-1008). Yet, and as illustrated in the appended examples, modifications of the published sequences of MT413 may also be employed in the context of this invention. For example, it is also envisaged that a nucleotide sequence encoding for an anti-CD4 (VH) part be employed which comprises a coding sequence as follows: and which encodes a amino acid sequence as follows:

The DNA encoding a modified (VL) of MT413 and being illustratively employed in the appended examples comprises the following nucleotide sequence:

This DNA encodes for an amino acid sequence as shown herein below:

In a most preferred embodiment of the present invention, the chemokine construct is a construct, wherein said (VH) anti-CD4 has at least one CDR3 comprising the amino acid sequence: has at least one CDR2 comprising the amino acid sequence : and/or has at least one CDR1 comprising the amino acid sequence :

Preferably, the DNA sequence encoding said (VH) anti-CD4 has at least one CDR3 comprising the nucleic acid sequence: has at least one CDR2 comprising the nucleic acid sequence : and/or has at least one CDR1 comprising the nucleic acid sequence:

Furthermore, the present invention provides for chemokine, wherein the (VL) anti-CD4 as defined herein above has at least one CDR3 comprising the amino acid sequence has at least one CDR2 comprising the amino acid sequence : and/or has at least one CDR1 comprising the amino acid sequence :

Preferably, the DNA sequence encoding the (VL) anti-CD4 as defined herein above has at least one CDR3 compmsing the nucleic acid sequence: has at least one CDR2 comprising the nucleic acid sequence : and/or
has at least one CDR1 comprising the nucleic acid sequence

Most preferably, the scFv anti-CD4 part of the chemokine constructs of the present invention does not only comprise at least one of the CDRs as defined herein above, but comprises two, and still most preferred three CDRs, i.e. the corresponding CDRs1, CDRs2 and CDRs3.

Most preferably, the invention relates to a chemokine construct as defined herein above, wherein said RANTES or RANTES fragment is a RANTES comprising an amino acid sequence an amino acid sequence Ser Pro Tyr Ser Ser Asp Thr Thr Pro Cys Cys Phe Ala Tyr Ile Ala Arg Pro Leu Pro Arg Ala His Ile Lys Glu Tyr Phe Tyr Thr Ser Gly Lys Cys Ser Asn Pro Ala Val Val Phe Val Thr Arg Lys Asn Arg Gin Val Cys Ala Asn Pro Glu Lys Lys Trp Val Arg Glu Tyr Ile Asn Ser Leu Gln Met Ser (SEQ ID No. 16; RANTES comprising an amino acid exchange from glutamic acid to glutamine at position 66);
an amino acid sequence
Tyr Ser Ser Asp Thr Thr Pro Cys Cys Phe Ala Tyr Ile Ala Arg Pro Leu Pro Arg Ala His Ile Lys Glu Tyr Phe Tyr Thr Ser Gly Lys Cys Ser Asn Pro Ala Val Val Phe Val Thr Arg Lys Asn Arg Gln Val Cys Ala Asn Pro Glu Lys Lys Trp Val Arg Glu Tyr Ile Asn Ser Leu Glu Met Ser (SEQ ID No. 22; functional RANTES fragment, whereby the first two amino acids have been deleted);
or an amino acid sequence
Tyr Ser Ser Asp Thr Thr Pro Cys Cys Phe Ala Tyr Ile Ala Arg Pro Leu Pro Arg Ala His Ile Lys Glu Tyr Phe Tyr Thr Ser Gly Lys Cys Ser Asn Pro Ala Val Val Phe Val Thr Arg Lys Asn Arg Gln Val Cys Ala Asn Pro Glu Lys Lys Trp Val Arg Glu Tyr Ile Asn Ser Leu Gln Met Ser (SEQ ID No. 24; RANTES comprising an amino acid exchange from glutamic acid to glutamine at position 66 and comprising a deletion of two amino acids at the N-terminus).

Preferably, the RANTES to be employed as functional part in the chemokine construct of the present invention is encoded by a DNA comprising the nucleic acid sequence the nucleic acid sequence: the nucleic acid sequence : or
the nucleic acid sequence

As illustrated in the appended examples, chemokine constructs comprising modified and/or mutated RANTES are still functional in accordance with this invention. For example, RANTES comprising an N-terminal deletion of two amino acids may be employed. Yet, even a deletion of three amino acids in the RANTES part leads to a functional, inventive chemokine construct. The constructs of the invention, as well as modified constructs comprising, inter alia, functional fragments of RANTES may be tested for the functionality by assays described herein above and in the appended examples. Furthermore, a chemokine construct of the present invention may comprise modified RANTES molecule, like the Leu-RANTES (Polo et al., 2000, Eur. J. Immunol. 30, 3190-3198),

The linker to be used in the chemokine construct of the invention has been described above. Preferably, the invention relates to a chemokine construct, wherein said linker between (VH) anti-CD4 or (VL) anti-CD4 and RANTES comprises between 3 and 30 amino acids and more preferably between 5 and 15 amino acids. The polypeptide linker between (VH) anti-CD4 and (VL) anti-CD4 comprises preferably between 5 and 30 amino acids, preferably between 7 and 20 amino acids, more preferably between 8 and 20 amino acids, more preferably between and more preferably between 8 and 18 amino acids and most preferably between 13 and 17 amino acids. Preferably, a hydrophilic, flexible peptide linker may be employed in the chemokine construct of the invention. Preferred amino acids are in this context: Gly. Ser, Thr and Val.

Linkers to be employed in the chemokine constructs comprise preferably the amino acid sequence Gly Gly Gly Gly Ser (SEQ ID NO.:51). Yet, as discussed herein above and as illustrated in the appended examples, said linker may also comprise multimers of said Gly/Ser sequence, like, (Gly₄Ser)₂ (SEQ ID NO.:53).

In accordance with the present invention, the inventive chemokine construct may also (further) comprise a tag. These tags are particularly useful in purification processes after synthesis of the inventive constructs or may be employed as labels. Such tags comprise Myc-tags, FLAG-tags, HA-tags, HIS-tag and the like. Preferably, these tags have an amino acid length in the range of one to thirty amino acids.

In a most preferred embodiment, the present invention relates to a chemokine construct comprising an amino acid sequence or

Preferably, the inventive chemokine construct is encoded by a nucleic acid sequence or by a nucleic acid molecule comprising the following nucleotide sequence: or

The invention also provides for a chemokine construct, wherein said chemokine construct as defined herein above further comprises an additional cytokine or chemokine linked to the scFv anti-CD4 part. Preferred in this context are, inter alia, chemokines which are capable of binding to HIV co-receptors, like, CXCR4 or CMV-US 28. Most primary, clinical isolates of primate immunodeficiency viruses use CCR5 for entry (Feng et al., Science 272, 872-877 (1996); Choe et al., Cell 85, 1135-1148 (1996); Deng et al, Nature 381, 661-666 (1996); Dragic et al., Nature 381, 667-673 (1996); Doranz et al., Cell 85, 1149-1158 (1996); Alkhatib et al., Science 272, 1955 -1958 (1996)). For most HIV-1 isolates that are transmitted and that predominate during the early years of infection, CCR5 is an obligate coreceptor, and rare individuals that are genetically deficient in CCR5 expression are relatively resistant to HIV-1 infection (Connor et al., J. Exp. Med. 185, 621-628 (1997); Zhang et al., Nature 383, 768 (1996); Bjömdal et al., J. Virol. 71, 7478-7487 (1997); Dean et al., Science 273, 1856-1862 (1996); Liu et al., Cell 86, 367-377 (1996); Paxton et al., Nature Med. 2, 412-417 (1996); Samson et al., Nature 382, 722-725 (1996)). HIV-1 isolates arising later in the course of infection often use other chemokine receptors, frequently CXCR4, in addition to CCR5. Specific further co-receptors, in accordance with the present invention comprise, but are not limited to, CXCR3, CXCR4, CXCR5, CCR1, CCR2, CCR3, CCR4, CCR5, CCR6, CCR7, CCR8, CCR9, XCR1, CCR10 and CX3CR1. Chemokines and/or chemokine ligands binding to said receptors are well known in the art and shown, inter alia, disclosed in Murphy et al. (2000), Pharm. Reviews 52, 145-176. The chemokines/ligands, are preferably primate, more preferably human chemokines/ligands.

Accordingly,_these chemokines or "receptor ligands" comprise, e.g. for CCR2 the chemokines MIP1alpha, MCP-1 and MCP-2, for CCR3 the chemokines MCP-2, eotaxin, eotaxin3, eotaxin4, LKN1, MPIF-2 and LD78beta, for CCR5 the chemokines RANTES, MIP1alpha, MIP1beta, MCP-2 and LD78beta, for CCR8 the chemokines I-309, MIP1beta and TARC, for CXCR6 the ligand CXCL16, for CX3CR1 the fractalkine and for CMV-US28 the MCP-1, MIP1alpha and MIP1beta. Yet, it is also envisaged that a further RANTES or a functional fragment thereof may be linked to the scFv anti-CD4 part of the present invention. In accordance with this invention, also truncated and or modified versions of these ligands/chemokines may be employed in the inventive "double chemokine construct" described herein. A particularly preferred additional chemokine to be linked to the scFv anti-CD4 part is SDF-1 or a functional fragment thereof. SDF-1 is the ligand,which is capable of binding to the HIV co-receptor CXCR4, used by T-tropic strains of HIV-1. SDF-1 exists in different forms, an alpha and a beta-form, which both may be employed in accordance with this invention.

Accordingly, the present invention furthermore provides for a chemokine construct which has the structural domain form of (a) RANTES-linker-(VH) anti-CD4-linker-(VL) anti-CD4-linker-SDF-1; (b) SDF1-linker-(VL) anti-CD4-linker-(VH) anti-CD4 -linker-RANTES, or (c) SDF1-linker-(VH) anti-CD4-linker-(VL) anti-CD4 -linker-RANTES.

The invention also relates to a polynucleotide, which upon expression encodes a chemokine construct as defined herein. Preferably said polynucleotide is (a) a polynucleotide comprising the nucleic acid molecule in particular encoding the polypeptide as depicted in SEQ ID NO: 18, SEQ. ID NO: 20 or SEQ ID NO: 30; (b) a polynucleotide comprising the nucleic acid molecule as depicted in SEQ ID NO: 17, SEQ ID NO: 19 or SEQ ID NO: 29; or (c) a polynucleotide hybridizing under stringent conditions to the complementary strand of a polynucleotide of (a) or (b).

With respect to the polynucleotides/nucleotide sequences characterized under (c) above, the term "hybridizing" in this context is understood as referring to conventional hybridization conditions, preferably such as hybridization in 50% formamide/6xSSC/0.1%SDS and 100µg/ml ssDNA, in which temperatures for hybridization are above 37°C and temperatures for washing in 0.1xSSC/0.1%SDS are above 55°C. Most preferably, the term "hybridizing" refers to stringent hybridization conditions, for example such as described in Sambrook et al., "Molecular Cloning: A Laboratory Manual", Cold Spring Harbor Laboratory Press, Cold Spring Harbor, New York, 1989. It is envisaged that the polynucleotides characterized under (c) above are highly homologous to the polynucleotides as defined in (a) and/or (b) and comprise a homology of at least 95%, more preferably of at least 97%, and most preferably 99% with the polynucleotides of (a) and/or (b).

Polynucleotides as defined and characterized under (c), therefore may encode for polypeptides being highly homologous to the polypeptides as defined in (a) and (b). The person skilled in the art can easily test the capacity of such homologous polypeptides to internalize CCR5, for example, on cells which are infected by a primate immunodeficiency virus, like HIV-1 or target cells involved in immunological disorders or disclosed herein. The person skilled in the art can easily adopt the in vitro, in vivo and ex vivo experiments of the appended examples to verify the binding and/or depletion properties of such constructs.

Furthermore, said polynucleotide/nucleic acid molecule may contain, for example, thioester bonds and/or nucleotide analogues. Said modifications may be useful for the stabilization of the nucleic acid molecule against endo- and/or exonucleases in the cell. Said nucleic acid molecules may be transcribed by an appropriate vector containing a chimeric gene which allows for the transcription of said nucleic acid molecule in the cell.

The polynucleotide/nucleic acid molecule of the composition of the present invention may be a recombinantly produced chimeric nucleic acid molecule comprising any of the afore mentioned nucleic acid molecules either alone or in combination.

According to a further embodiment, the present invention relates to polynucleotides,which are fused to suitable expression control sequences known in the art to ensure proper transcription and translation of the polypeptide. Said polynucleotides may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector.

A preferred embodiment of the invention relates to a vector comprising the afore mentioned polynucleotide.

The term "vector" defines according to the definition known in the art a vehicle by which a foreign nucleotide can be transferred into an organism.

Preferably said vectors are expression vectors which enable expression of a peptide encoded by said polynucleotide.

Such vectors may comprise further genes such as marker genes which allow for the selection of said vector in a suitable host cell and under suitable conditions. Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in prokaryotic host cells comprise, e.g., the PL, lac, trp or tac promoter in E. coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polypeptide/chemokine-construct of the invention and are well known in the art; see also, e.g., the appended examples. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an N-terminal identification peptide imparting desired characteristics, e.g., stabilization or simplified purification of expressed recombinant product; see supra. In this context, suitable expression vectors are known in the art such as Okayama-Berg cDNA expression vector pcDV1 (Pharmacia), pCDM8, pRc/CMV, pcDNA1, pcDNA3 (In-vitrogene), or pSPORT1 (GIBCO BRL).

Preferably, the expression control sequences will be eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptides/chemokine-constructs of the invention may follow; see, e.g., the appended examples.

As described above, the polynucleotide encoding the polypeptide/chemokine-construct of the invention can be used alone or as part of a vector to express the polypeptide of the invention in cells, for, e.g., gene therapy to treat diseases related to HIV infection. The polynucleotides or vectors containing the DNA sequence(s) encoding any one of the above described polypeptides is introduced into the cells which in turn produce the polypeptide of interest. Gene therapy, which is based on introducing therapeutic genes into cells by ex-vivo or in-vivo techniques is one of the most important applications of gene transfer. Suitable vectors, methods or gene-delivery systems for in-vitro or in-vivo gene therapy are described in the literature and are known to the person skilled in the art; see, e.g., Giordano et al., Nature Medicine 2 (1996), 534-539; Schaper & Ito, Circ. Res. 79 (1996), 911-919; Anderson, Science 256 (1992), 808-813; Verma, Nature 389 (1994), 239; Isner et al., Lancet 348 (1996), 370-374; Muhlhauser et al., Circ. Res. 77 (1995), 1077-1086; Onodera et al., Blood 91 (1998), 30-36; Verma et al., Gene Ther. 5 (1998),692-699; Nabel et al., Ann. N.Y. Acad. Sci. 811 (1997), 289-292; Verzeletti et al., Hum. Gene Ther. 9 (1998), 2243-51; Wang & Finer, Nature Medicine 2 (1996), 714-716; WO 94/29469; WO 97/00957, US 5,580,859; US 5,589,466; or Schaper & Ito, Current Opinion in Biotechnology 7 (1996), 635-640, and references cited therein. The polynucleotides and vectors of the invention may be designed for direct introduction or for introduction via liposomes, or viral vectors (e.g., adenoviral, retroviral) into the cell. Preferably, said cell is a germ line cell, embryonic cell, or egg cell or derived therefrom, most preferably said cell is a stem cell. An example for an embryonic stem cell can be, inter alia, a stem cell as described in, Nagy et al., Proc. Natl. Acad. Sci. USA 90 (1993), 8424-8428.

In accordance with the above, the present invention relates to vectors, particularly plasmids, cosmids, viruses and bacteriophages used conventionally in genetic engineering that comprise a polynucleotide encoding a polypeptide/chemokine-construct of the invention. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell populations. Methods which are well known to those skilled in the art can be used to construct recombinant vectors; see, for example, the techniques described in Sambrook et al., Molecular Cloning: A Laboratory Manual, Cold Spring Harbor Laboratory (1989) N.Y. and Ausubel, Current Protocols in Molecular Biology, Green Publishing Associates and Wiley Interscience, N.Y. (1989). Alternatively, the polynucleotides and vectors of the invention can be reconstituted into liposomes for delivery to target cells. The vectors containing the polynucleotides of the invention can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook et al., supra. Once expressed, the polypeptides of the present invention can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure polypeptides of at least about 90 to 95% homogeneity are preferred, and 98 to 99% or more homogeneity are most preferred, for pharmaceutical uses. Once purified, partially or to homogeneity as desired, the polypeptides may then be used therapeutically (including extracorporeally) or in developing and performing assay procedures.

In a still further embodiment, the present invention relates to a cell containing the polynucleotide or vector described above. Accordingly, a particularly preferred embodiment of the invention relates to a cell transfected with the aforementioned polynucleotide. Preferably, said cell is a eukaryotic, most preferably a mammalian cell if, inter alia, therapeutic uses of the polypeptide are envisaged. Particularly preferred cells are CHO-cells, like CHO DHFR- cells, CHO K1 cells, X63 or HEK 293 cells. Yet, other cells than mammalian cells may be employed like insect cells, e.g. SP-2 cells. Of course, yeast and less preferred prokaryotic, e.g., bacterial cells may serve as well, in particular if the produced polypeptide is used as a research tool.

The polynucleotide or vector of the invention which is present in the host cell may either be integrated into the genome of the host cell or it may be maintained extrachromosomally.

The term "prokaryotic" is meant to include all bacteria which can be transformed or transfected with a DNA or RNA molecules for the expression of a polypeptide of the invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host employed in a recombinant production procedure, the polypeptides of the present invention may be glycosylated or may be non-glycosylated. Polypeptides of the invention may also include an initial methionine amino acid residue. A polynucleotide coding for a polypeptide of the invention can be used to transform or transfect the host using any of the techniques commonly known to those of ordinary skill in the art. Especially preferred is the use of a plasmid, a vector or a virus containing the coding sequence of the polypeptide of the invention and genetically fused thereto an N-terminal FLAG-tag and/or C-terminal His-tag. Preferably, the length of said FLAG-tag is about 4 to 8 amino acids, most preferably 8 amino acids. Methods for preparing fused, operably linked genes and expressing them in, e.g., mammalian cells and bacteria are well-known in the art (Sambrook et al., Molecular Cloning: A Laboratory Manual, Cold Spring Harbor Laboratory, Cold Spring Harbor, N.Y., 1989). The genetic constructs and methods described therein can be utilized for expression of the polypeptide of the invention in eukaryotic or prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. Furthermore, transgenic animals, preferably mammals, comprising cells of the invention may be used for the large scale production of the polypeptide of the invention.

In a further embodiment, the present invention thus relates to a method for the preparation of the chemokine construct described above comprising cultivating an above described cell of the invention under conditions suitable for the expression of the polypeptide and isolating the construct from the cell or the culture medium.

The transformed hosts can be grown in fermentors and cultured according to techniques known in the art to achieve optimal cell growth. The polypeptide of the invention can then be isolated from the growth medium, cellular lysates, or cellular membrane fractions. The isolation and purification of the, e.g., (microbially) expressed polypeptides/chemokine-constructs of the invention may be by any conventional means such as, for example, preparative chromatographic separations and immunological separations such as those involving the use of monoclonal or polyclonal antibodies directed, e.g., against a tag of the polypeptide/chemokine-construct of the invention or as described in the appended examples.

Thus, the present invention allows the recombinant production of polypeptides/chemokine-constructs comprising binding sites having affinity and specificity for an epitope of the CD4 and CCR5 antigen (receptor for RANTES), respectively, and optionally a further functional.

Depending on the host cell, renaturation techniques may be required to attain proper conformation. If necessary, point substitutions seeking to optimise binding may be made in the DNA using conventional cassette mutagenesis or other protein engineering methodology such as is disclosed herein. Preparation of the polypeptides of the invention may also be dependent on knowledge of the amino acid sequence (or corresponding DNA or RNA sequence) of bioactive proteins such as enzymes, toxins, growth factors, cell differentiation factors, receptors, antimetabolites, hormones or various cytokines or lymphokines. Such sequences are reported in the literature and available through computerised data banks.

Furthermore, the constructs of the invention can be used in the management of immunological disorders, in particular autoimmune diseases, allergic diseases, inflammatory diseases and AIDS (HIV-infection), as documented in the appended examples.

Accordingly, the present invention provides for compositions comprising the aforementioned polypeptide(s)/chemokine-construct(s), the polynucleotide or the vector of the invention.

The term "composition", in context of this invention, comprises at least one polynucleotide, vector, host, antibody construct and/or chemokine construct as described herein. Said composition, optionally, further comprises other molecules, either alone or in combination, like e.g. molecules which are capable of modulating and/or interfering with the immune system. The composition may be in solid, liquid or gaseous form and may be, inter alia, in a form of (a) powder(s), (a) tablet(s), (a) solution(s) or (an) aerosol(s). In a preferred embodiment, said composition comprises at least two, preferably three, more preferably four, most preferably compounds as described in the invention.

Preferably, said composition is a pharmaceutical composition further comprising, optionally, a pharmaceutically acceptable carrier, diluent and/or excipient.

The term "pharmaceutical composition", as used in accordance with the present invention, comprises at least the compound as identified herein above, such as a polypeptide/chemokine-construct, optionally, further molecules, either alone or in combination, e.g., molecules like cytokines, chemokines, immunoglobulins, or lymphokines, optionally, adjuvants.

Examples of suitable pharmaceutical carriers are well known in the art and include phosphate buffered saline solutions, water, emulsions, such as oil/water emulsions, various types of wetting agents, sterile solutions, etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. Intravenous administration is particularly preferred. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. However, a more preferred dosage for continuous infusion might be in the range of 0.01 µg to 10 mg units per kilogram of body weight per hour. Particularly preferred dosages are recited herein below. Progress can be monitored by periodic assessment. Dosages will vary but a preferred dosage for intravenous administration of DNA is from approximately 10⁶ to 10¹² copies of the DNA molecule. The compositions of the invention may be administered locally or systematically. Administration will generally be parenterally, e.g., intravenously; yet external administration is also envisaged. DNA may also be administered directed to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition of the present invention might comprise proteinaceous carriers, like, e.g., serum albumin or immunoglobuline, preferably of human origin. Furthermore, it is envisaged that the pharmaceutical composition of the invention might comprise further biologically active agents, depending on the intended use of the pharmaceutical composition. Such agents might be drugs acting on the immunological system, drugs used in anti-viral treatment, in particular in HIV-treatment (for example, HAART) and AIDS management and/or anti-inflammatory drugs. It is, for example, envisaged that patients are treated as early as possible with HAART until viral load is below detection level for several weeks to months. Early treatment of infected patients with HAART prevents the transition of viral strains from usage of CCR5 to other chemokine receptors, like CXCR4 (Connor et al., (1997) J. Exp. Med. 185, 621-628). Constructs as disclosed in the present invention can be administered in addition to HAART intravenously, subcutaneously, and/or into the cerebral-spinal fluid. Other agents for combination with the inventive constructs could comprise, inter alia, bispecific antibodies as described in Brühl at al. (J. Immunol. 2001, 166, 2420-2426).

It is envisaged by the present invention that the various polynucleotides and vectors of the invention are administered either alone or in any combination using standard vectors and/or gene delivery systems, and optionally together with a pharmaceutically acceptable carrier or excipient. Subsequent to administration, said polynucleotides or vectors may be stably integrated into the genome of the subject. Preferably said subject is a human. On the other hand, viral vectors may be used which are specific for certain cells or tissues and persist in said cells. Suitable pharmaceutical carriers and excipients are well known in the art. The pharmaceutical compositions prepared according to the invention can be used for the prevention or treatment or delaying of different kinds of immunological diseases, which may be related to inflammation, in particular inflammatory bowel diseases, inflammatory renal diseases, inflammatory joint diseases like (chronic) arthritis. Furthermore, the pharmaceutical composition of the present invention may be employed to eliminate cells which are latently infected with a virus, preferably a primate immunodeficiency virus, more preferably with HIV(-1). Said HIV infections comprise particular infections of T cell topic and dual topic variants of HIV.

A therapeutically effective dose refers to that amount of compound which ameliorate the symptoms or condition. Therapeutic efficacy and toxicity of such compounds can be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, LD₅₀/ED₅₀.

Furthermore, it is possible to use the polynucleotide or the vector of the invention for the preparation of compositions for gene therapy.

Suitable gene delivery systems may include liposomes, receptor-mediated delivery systems, naked DNA, and viral vectors such as herpes viruses, retroviruses, adenoviruses, and adeno-associated viruses, among others. Delivery of nucleic acids to a specific site in the body for gene therapy may also be accomplished using a biolistic delivery system, such as that described by Williams and coworkers (Proc. Natl. Acad. Sci. USA 88 (1991), 2726-2729). Further methods for the delivery of nucleic acids comprise particle-mediated gene transfer as, e.g., described in Verma et al., Gene Ther.15 (1998), 692-699.

It is to be understood that the introduced polynucleotides and vectors express the gene product after introduction into said cell and preferably remain in this status during the lifetime of said cell. For example, cell lines which stably express the polynucleotide under the control of appropriate regulatory sequences may be engineered according to methods well known to those skilled in the art. Rather than using expression vectors which contain viral origins of replication, host cells can be transformed with the polynucleotide of the invention and a selectable marker, either on the same or separate plasmids. Following the introduction of foreign DNA, engineered cells may be allowed to grow for 1-2 days in an enriched media, and then are switched to a selective media. The selectable marker in the recombinant plasmid confers resistance to the selection and allows for the selection of cells having stably integrated the plasmid into their chromosomes and grow to form foci which in turn can be cloned and expanded into cell lines.

A number of selection systems may be used, including but not limited to, the herpes simplex virus thymidine kinase (Wigler et al, Cell 11 (1977), 223-232), hypoxanthine-guanine phosphoribosyltransferase (Szybalska, Proc. Natl. Acad. Sci. USA 48 (1962), 2026), and adenine phosphoribosyltransferase (Lowy et al, Cell 22 (1980), 817-823) in tk⁻, hgprt⁻ or aprt⁻ cells, respectively. Also, antimetabolite resistance can be used as the basis of selection for dhfr, which confers resistance to methotrexate (Wigler et al., Proc. Natl. Acad. Sci. USA 77 (1980), 3567-3570; O'Hare et al., Proc. Natl. Acad. Sci. USA 78 (1981), 1527-1531), gpt, which confers resistance to mycophenolic acid (Mulligan & Berg, Proc. Natl. Acad. Sci. USA 78 (1981), 2072-2076); neo, which confers resistance to the aminoglycoside G-418 (Colbere-Garapin et al., J. Mol. Biol. 150 (1981), 1-14); hygro, which confers resistance to hygromycin (Santerre et al., Gene 30 (1984), 147-156); or puromycin (pat, puromycin N-acetyl transferase). Additional selectable genes have been described, for example, trpB, which allows cells to utilize indole in place of tryptophan, hisD, which allows cells to utilize histinol in place of histidine (Hartman & Mulligan, Proc. Natl. Acad. Sci. USA 85 (1988), 8047-8051); and ODC (omithine decarboxylase) which confers resistance to the ornithine decarboxylase inhibitor, 2-(difluoromethyl)-DL-ornithine, DFMO (McCologue, 1987, In: Current Communications in Molecular Biology, Cold Spring Harbor Laboratory ed.).

In a further embodiment, the present invention relates to the use a of a chemokine construct, a polynucleotide or a vector of the invention for the preparation of a pharmaceutical composition for the treatment of an HIV-infection or of AIDS.

Said anti-HIV treatment may comprise HAART. HAART therapy consists of a cocktail of three classes anti-viral drugs. The classes are nucleosidal reverse transcriptase inhibitors (NRTI), non-nucleosidal reverse transcriptase inhibitors (NNRTI) and protease inhibitors (PI). Usually 2 to 4 drugs from preferentially more than one class are combined to reduce viral load to almost non-detectable levels.

Said treatment of an immunological disorder may comprise anti-inflammatory agents and immunosuppressive agents. Anti-inflammatory agents may be selected from the group consisting of azathioprine, cyclophophamide, glucocorticoids like prednisone and corticosteroids. Immunosuppressive agents may comprise cyclosporin A, Tacrolimus (FK506), Sirolimus (Rapamycin). Protein drugs may comprise calcineurin, beta-interferon, anti-TNF alpha monoclonal antibodies (remicade). Dosing and use of anti-inflammatory agents and immunosuppressive agents is described, inter alia, in Fauci et al., sic. Further treatment options are known to the skilled artisan and, inter alia, described herein above.

In a further embodiment, the present invention relates to the use of a chemokine construct, a polynucleotide or a vector of the invention for the preparation of a pharmaceutical composition for the treatment of inflammatory diseases and/or autoimmune diseases, which comprise but are not limited to inflammatory renal disease, inflammatory bowel diseases, multiple sclerosis, skin diseases, allergic reactions, diabetes, transplant rejections and arthritis.

The invention relates also to a method for the production of a pharmaceutical composition comprising formulating the polypeptide/chemokine-construct, a polynucleotide or a vector of the invention in a pharmaceutically acceptable form. Furthermore, it is envisaged that said polypeptide/chemokine-construct might be modified by peptidomimetics. Methods for the generation and use of peptidomimetic combinatorial libraries are described in the prior art, for example Ostresh et al., Methods in Enzymology 267 (1996), 210-234, Domer et al., Bioorg. Med. Chem. 4 (1996), 709-715, Beeley, Trends Biotechnol. 12 (1994), 213-216, or al-Obeidi et al., Mol. Biotechnol. 9 (1998), 205-223.

The pharmaceutical composition of the invention may be administered to a patient by any appropriate method for the particular compound, e.g., orally, intravenously, parenterally, transdermally, transmucosally, or by surgery or implantation (e.g., with the compound being in the form of a solid or semi-solid biologically compatible and resorbable matrix) at or near the site where the effect of the compound is desired. Therapeutic doses are determined to be appropriate by one skilled in the art, see supra.

The invention further relates to a method of modifying a compound identified by the method of the invention as a lead compound to achieve (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

Moreover, the present invention relates to a method of producing a pharmaceutical composition comprising the step of formulating the polypeptide/chemokine-construct, a polynucleotide or a vector of the invention or refined according to the method as defined herein above with a pharmaceutically acceptable carrier and/or diluent.

The figures show

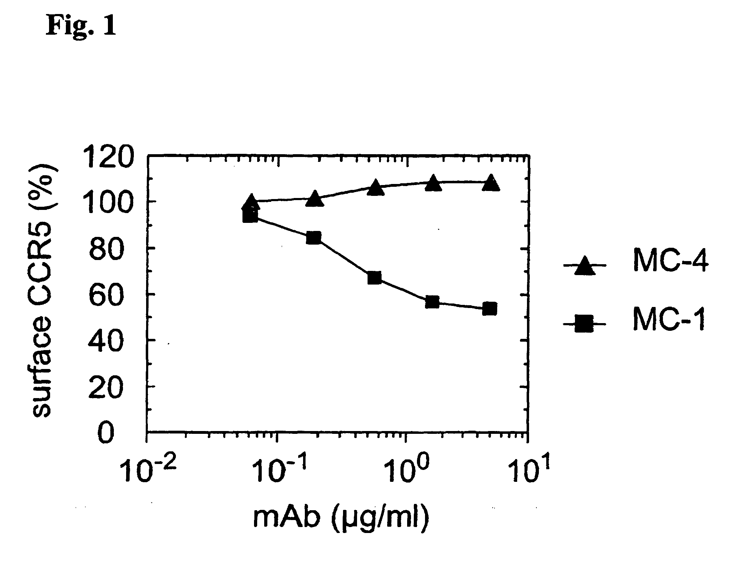

### Fig. 1: MC-1 Mab induces CCR5 endocytosis in CHO cells

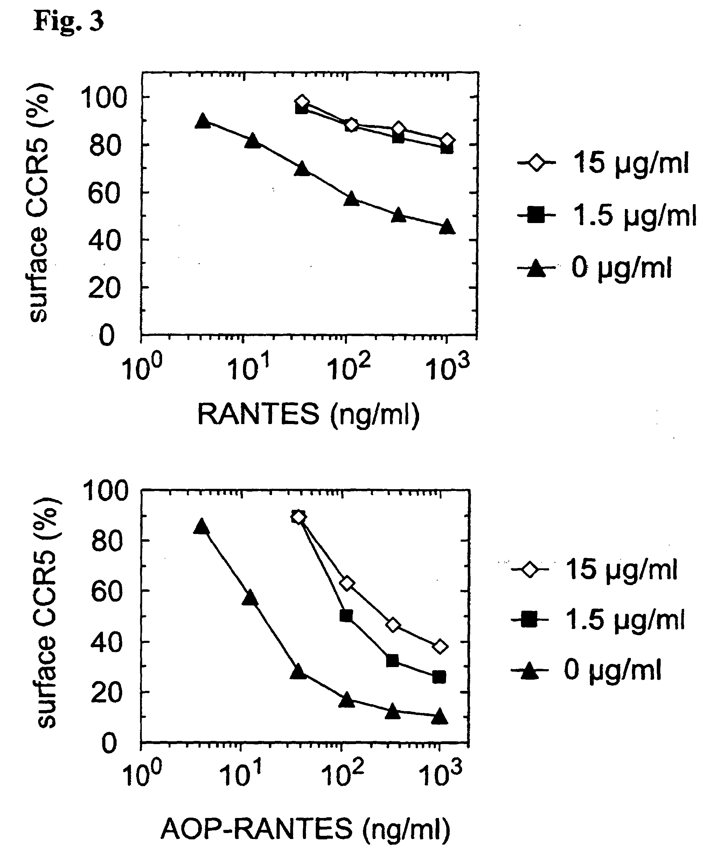

CHO cells expressing CCR5 were incubated with MC-1 and MC-4 for 30 minutes at 37°C. Cells were placed on ice, incubated with 15µg MC-1 or MC-4, washed with cold PBS and stained with FITC-conjugated anti-mouse Ig antibody. After washing cells were analyzed by flowcytometry. Cell surface CCR5 expression was detected by flow-cytometry using a saturating concentration of both antibodies for RANTES induced CCR5 downmodulation. MC-1 induced a 50 % decrease of cell surface CCR5 similar to RANTES on CHO cells (see Fig. 3) but had only a very weak effect on PBMC (not shown). Results were normalized for the fluorescence of unstimulated cells (100%) and for the background fluorescence (0%). All experiments were repeated at least twice with similar results.

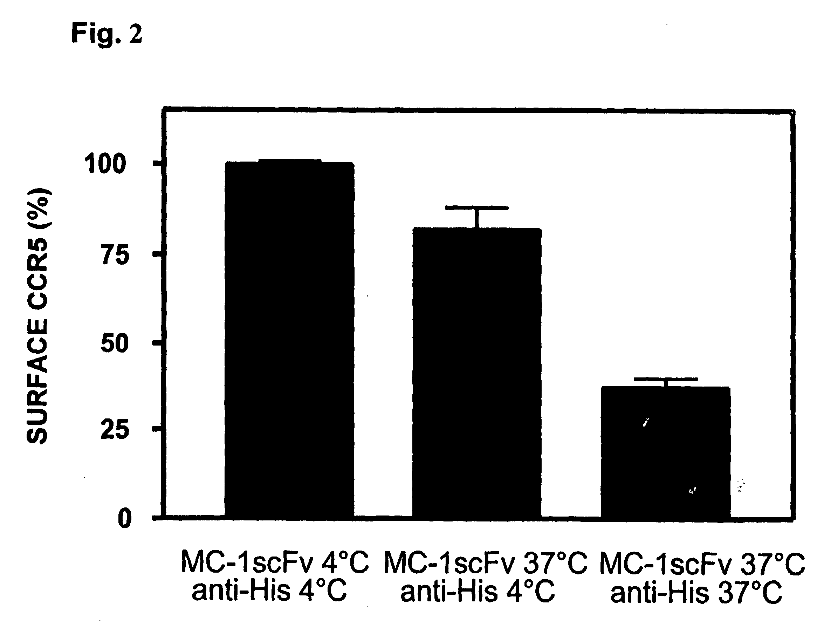

### Figure 2: Dimerization is required for MC-1 induced CCR5 endocytosis

CHO cells expressing CCR5 were incubated with scFv-MC-1 (3 µg/ml) for 45 minutes at 4°C or 37°C, washed twice with cold PBS and incubated with anti-His antibody at 4°C or 37°C as indicated. Surface expression of CCR5 was measured by flow cytometry with PE- labeled anti-mouse Ab. Fluorescence was normalized for cells incubated with scFv MC-1 and anti-His antibody both at 4°C (100%), and for background florescence (0%).

### Figure 3: MC-4 inhibits CCR5 endocytosis mediated by chemokines

CHO cells expressing CCR5 were pre-incubated with medium (0 µg/ml) or MC-4 (15 µg/ml or 1,5 µg/ml) for 20 minutes on ice and then incubated with RANTES or AOP-RANTES for 30 minutes at 37°C. Surface expression of CCR5 was measured by flow cytometry using the antibody MC-4 (15 µg/ml on ice) and FITC-conjugated secondary antibodies. Fluorescence was normalized as 100% in the absence of chemokines and 0% for background fluorescence. All experiments were repeated at least twice.

### Fig. 4: Scheme of the constructs RM and MR

Molecules combining a scFv anti-CD4 and RANTES in N- or C-terminal position.

### Fig. 5: Binding of RM and MR to CCR5 as shown by ligand competition assay

CHO cells stably transfected with CCR5 or CXCR4 were coincubated for 4 hours on ice with one of several inhibitors (RM, MR, RANTES or CD4 scFv) and 1 nM ¹²⁵I-RANTES. Relative binding of ¹²⁵I-RANTES is calculated as (specific binding [inhibitor]/specific binding [medium]). All incubations were performed in triplicates. Error bars indicate the standard deviation.

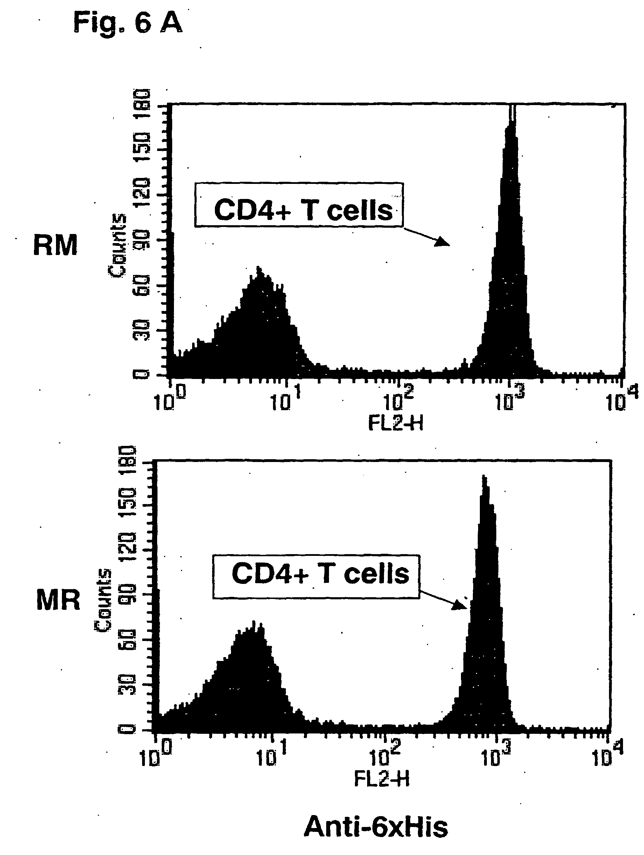

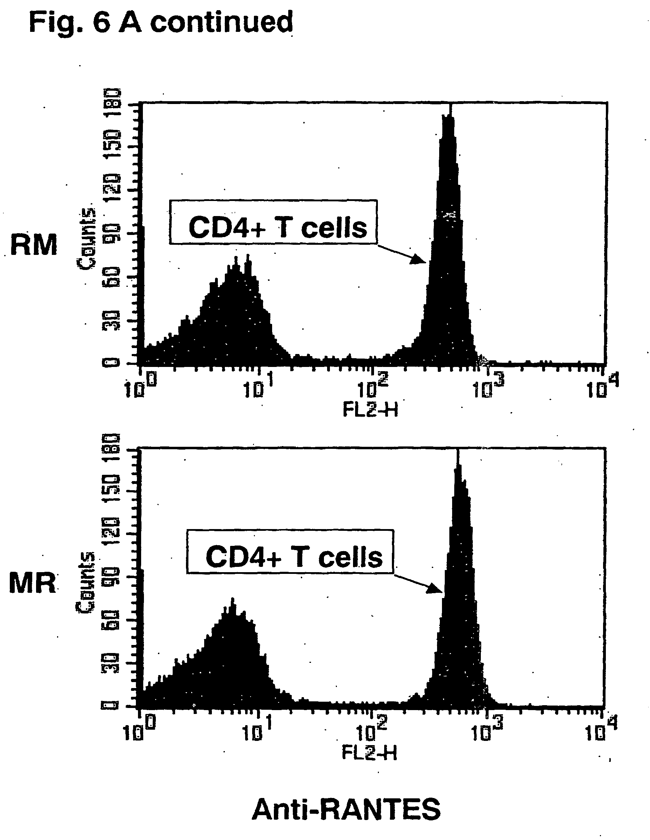

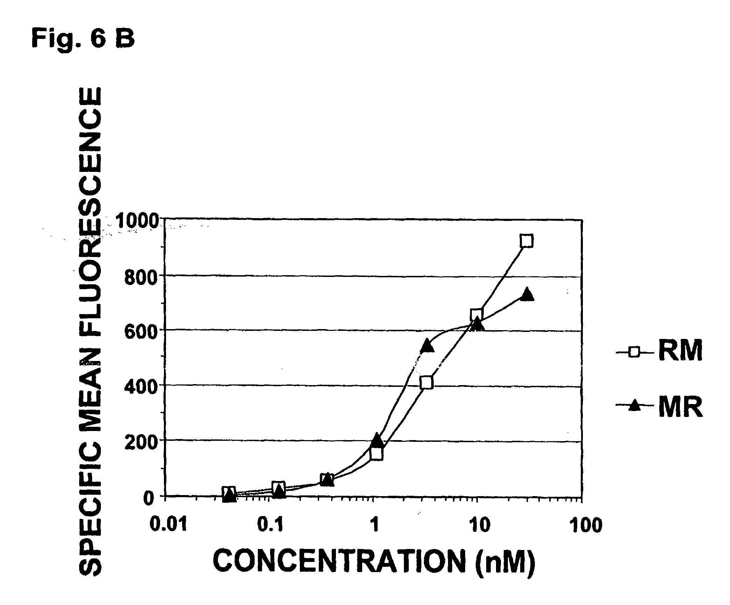

### Fig. 6: Binding of RM and MR to CD4 on T-cells

PBMC were incubated for 1h on ice with the constructs RM and MR in various concentrations. FACS analysis was performed with anti-6xHis antibody (Dianova) or an antibody against RANTES (VL-1) followed by incubation with PE-labelled rabbit-anti-mouse F(ab) fragments. Concentration dependent binding of RM and MR to CD4+ T cells is shown in Fig. 6B.

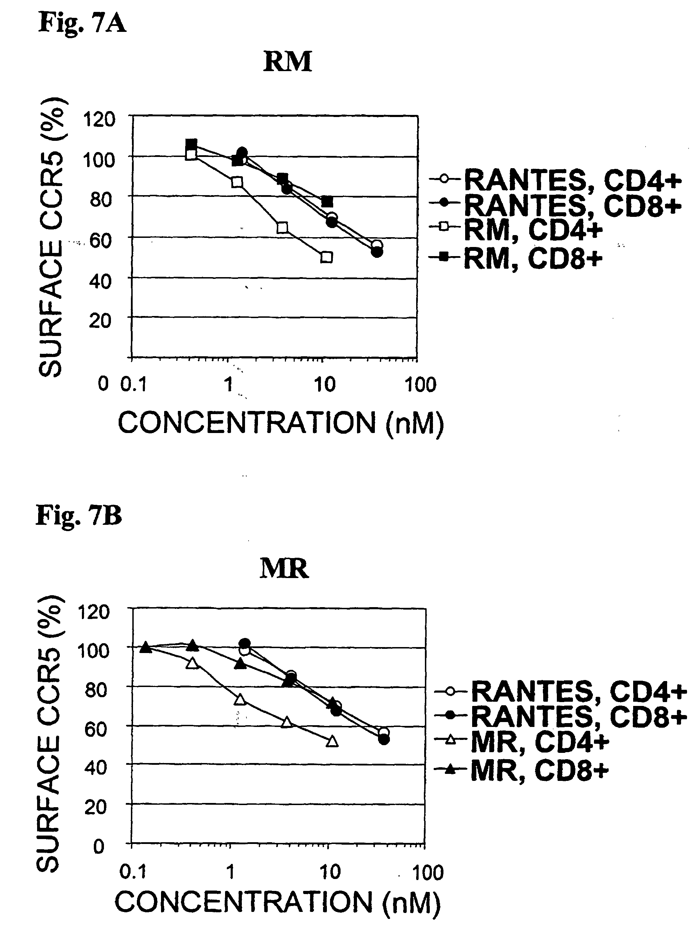

### Fig. 7: Simultaneous binding of RM and MR to CCR5 and CD4 on human T cells

RM and MR induced internalization of CCR5 on T cells is shown in Figure 7A and 7B, respectively. Human PBMC were incubated for 30 min at 37°C with various concentrations of RM (Fig. 7A), MR (Fig. 7B) and RANTES and stained on ice for 1 h with a combination of directly conjugated antibodies against CCR5, CD8 and CD4. - Mean channel fluorescence (MCF) of CCR5 was quantified on CD4 and CD8 positive T cells by FACS-analysis. Relative surface expression of CCR5 was calculated as [MCF(exp.)-MCF(negative control)]/[(MCF(medium)-MCF(negative control).

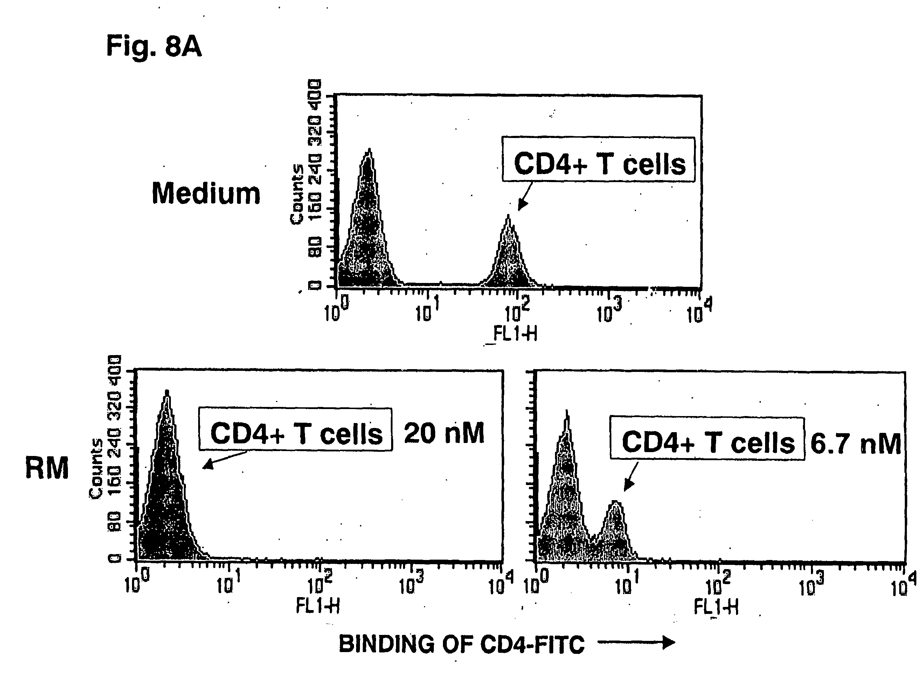

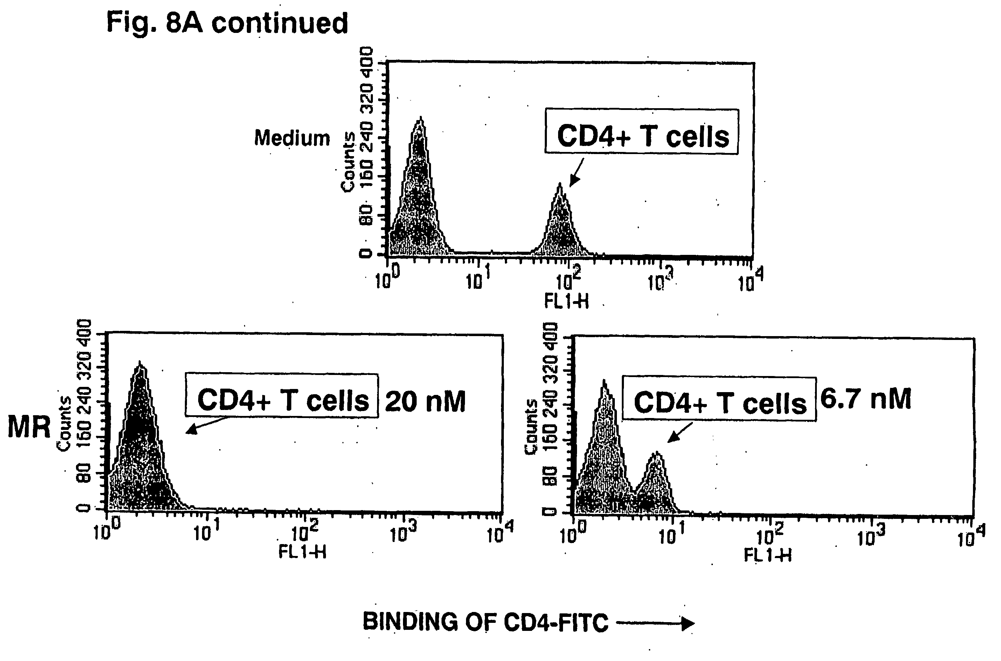

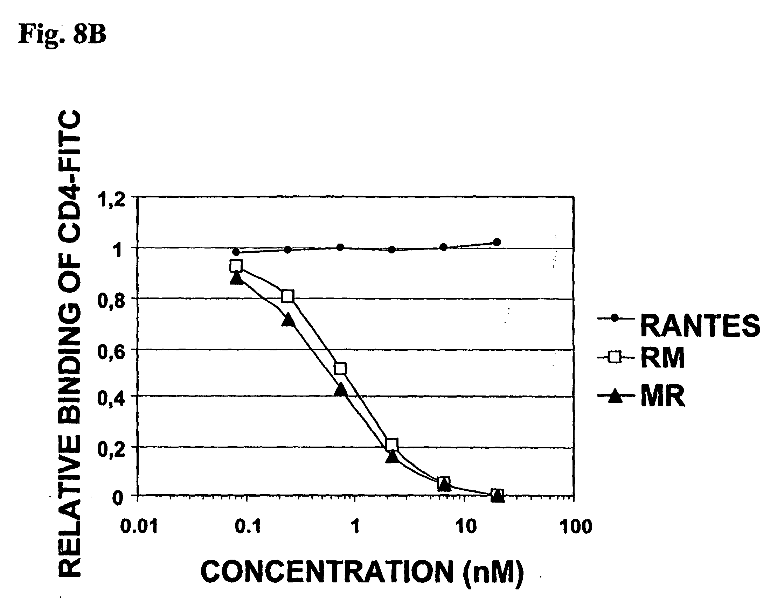

### Fig. 8: Binding of RM and MR to CD4 on T cells - Blockade of anti-CD4 mAb binding

Inhibition of CD4-FITC mAB binding to CD4+ T cells by RM or MR is shown in Fig. 8A. PBMC were preincubated with various concentrations of RM, MR or RANTES (RANTES not shown) for 1 h on ice. Without washing the cells CD4-FITC antibodies (Immunotech) were added and binding of CD4-FITC was detected by FACS analysis. The mean channel fluorescence (MCF) of CD4-FITC binding was quantified. Relative binding of CD4-FITC was calculated as [MCF(inhibitor)-MCF(negative control)]/[MCF{medium)-MCF(negative control) (Fig. 8A). Concentration dependent blockade of CD4 by RM and MR is shown in Fig. 8B.

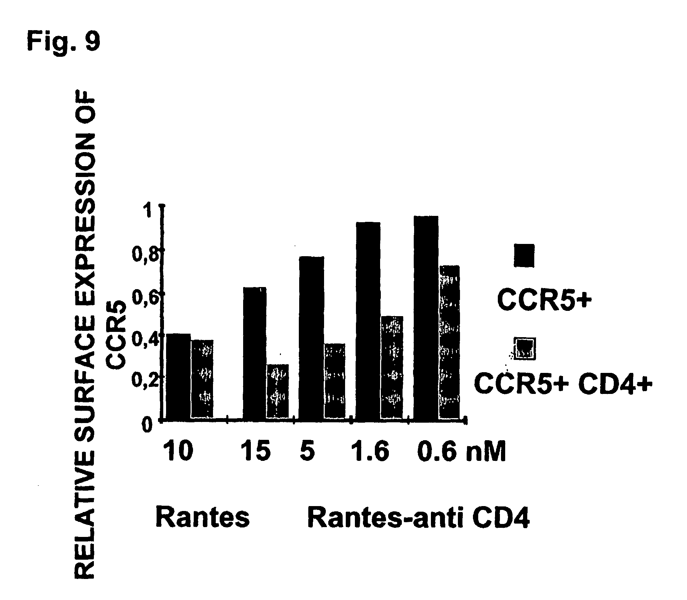

### Fig. 9: Downmodulation of CCR5 expression on CHO cells induced by RM

CHO cells expressing CCR5 or both CCR5 and CD4 were incubated for 30 min at 37°C with various concentrations of RANTES (10 nM or 15 nM) or RM (5 nM, 1,6 nM or 0,6 nM) or medium as control. Surface expression of CCR5 was measured by FACS analysis by staining cells with a rabbit antiserum against CCR5 and FITC labelled goat anti-rabbit antibody. Preimmune serum was used as negative control.

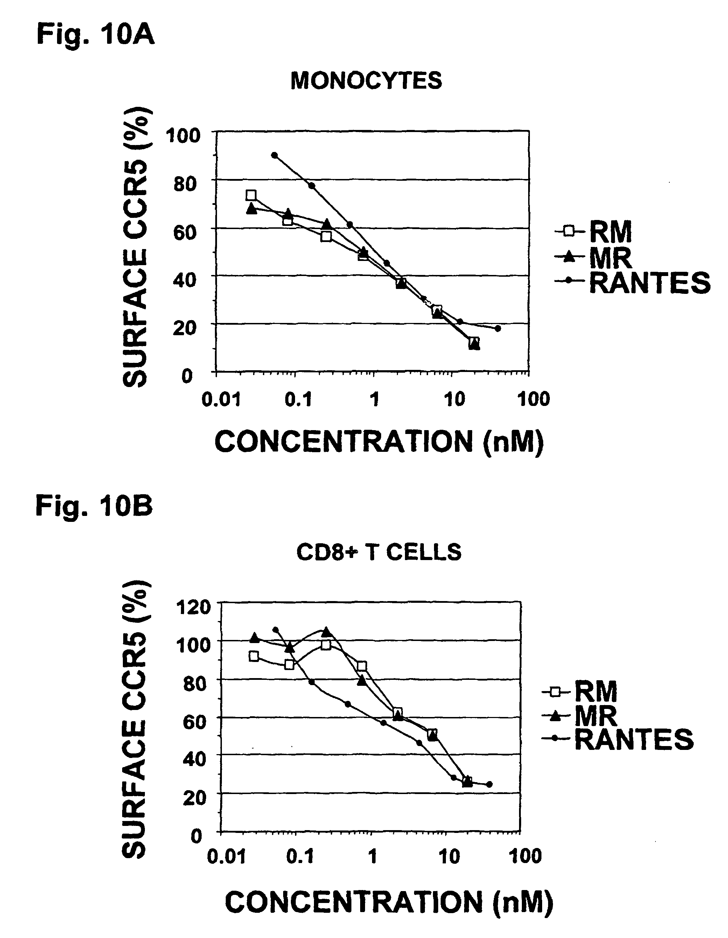

### Fig. 10: Binding of RM and MR to CCR5 as shown by internalization of CCR5

Internalization of CCR5 on monocytes and on CD8⁺ T cells is shown in Fig. 10A and Fig. 10B, respectively. Human PBMC were cultured overnight to induce expression of CCR5 on monocytes. Cells were incubated for 30 min at 37°C with various concentrations of RM, MR and RANTES and surface expression of CCR5 was then quantified by FACS analysis using antibody MC-1 (5µg/ml) followed by PE-conjugated rabbit anti-mouse F(ab)2 fragments. Cells were stained with directly conjugated CD8-Cychrome and CD14-APC antibodies (Immunotech). Monocytes and CD8+ T cells were identified by expression of CD14 and CD8 as well as by their light scatter properties.

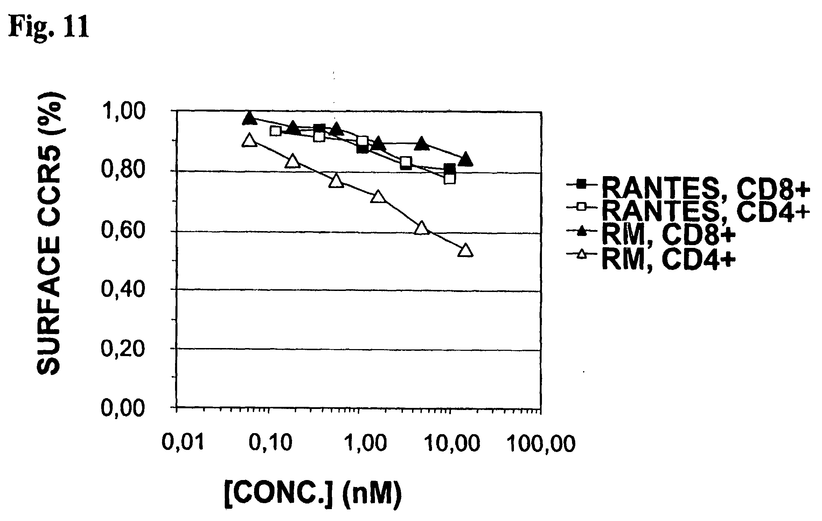

### Fig. 11: Internalization of CCR5 on human PBMC after washing out of chemokine constructs and RANTES

PBMC were preincubated for 30 min on ice with various concentrations of RANTES, RM and medium as control and then washed three times at 4°C. The cells were warmed up to 37°C and incubated for 30 min at 37°C. Surface expression of CCR5 was quantified on CD4+ and CD8+ T cells by FACS analysis by staining the cells with MC-1 or IgG1 isotype control and FITC labelled mAb against mouse IgG1. After washing cells were stained with Phycoerythrin-Cyanin5 labelled CD8 and Allo-Phycocyanin labelled CD3 antibodies. Lymphocytes were gated by their forward and sideward light scatter properties. CD8+ T cells were identified by expression of CD8 and CD3. CD4+ T cells were identified by expression of CD3 and absence of CD8.

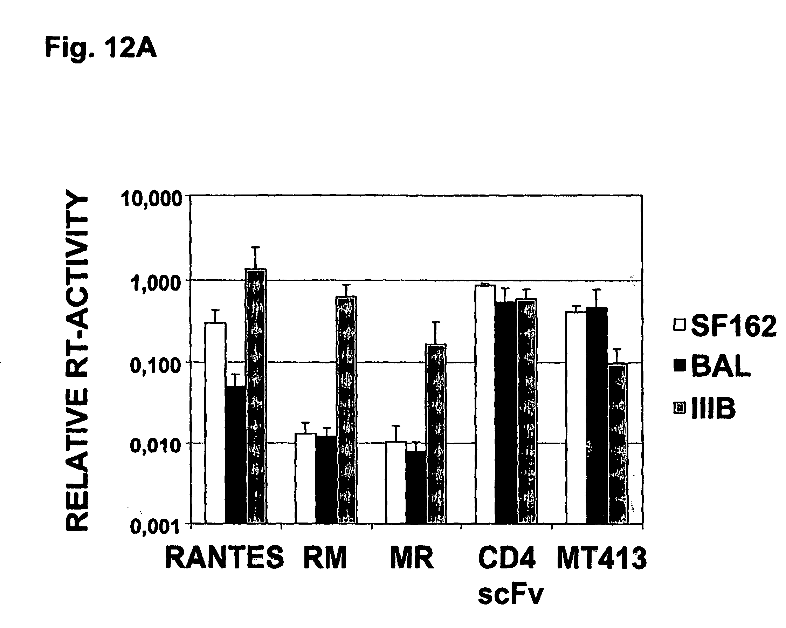

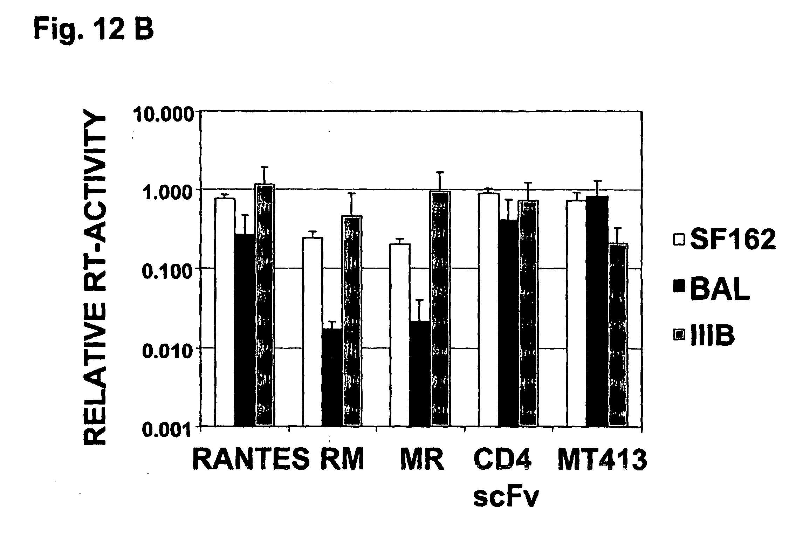

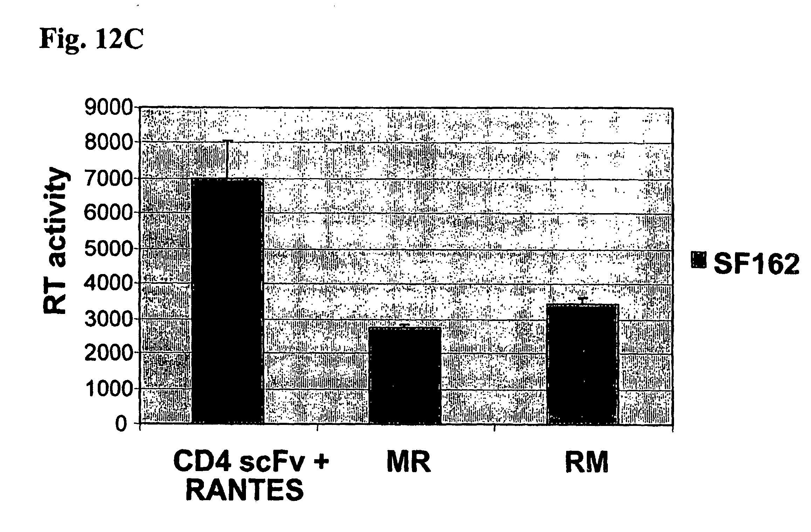

### Fig. 12: Blocking of infection with M-tropic HIV-1 strains by RM construct, MR construct and a mixture ofMR, RANTES and anti-CD4scFv

Isolated PBMC were cultured overnight with 20 U/ml IL-2. Cells were preincubated with RANTES or RM or MR at concentrations of 20 nM (Fig. 12A), 4 nM (Fig. 12B) or PBS as control. After 3 h HIV-1 virus was added. Two M-tropic strains SF162 and BAL were used as well as one T-tropic strain IIIB. RT-activity was measured after 8 days of infection. RT activity of PBMC not infected with HIV-1 was measured to obtain the negative control value. Relative RT activity was calculated as [RT(inhibitor) - RT(negative control)] : [RT (PBS control) - RT(negative control)]. Error bars indicate the standard deviation.

The HIV infection blocking activity of the MR or RM constructs was compared with the HIV infection blocking activity of the combination of RANTES and anti-CD4scFv (Fig. 12C). PBMC were isolated from full blood of healthy donors by ficoll density centrifugation and cultured overnight with 20 U/ml IL-2. Cells were preincubated with inhibitors at concentrations of 20 nM each. After 3 h the M-tropic HIV-1 virus SF 162 was added. After incubation for 9 days RT-activity was measured with a commercial kit according to manufactures recommendations. All incubations were performed in quadruplicates. Error bars indicate the standard deviation.

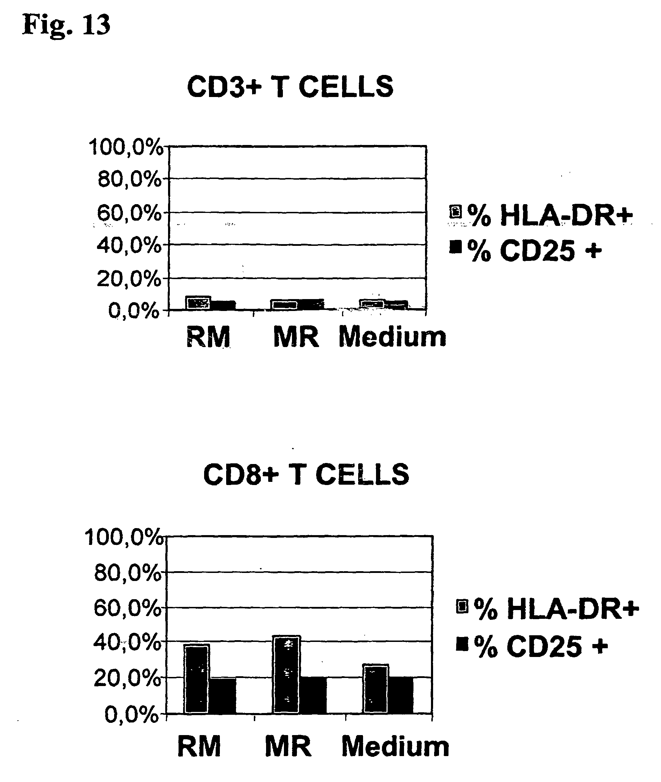

### Fig. 13: RM and MR do not inhibit MLR

PBMC from two different donors were co-incubated for 14 days either in medium alone or in medium containing RM (10 nM) or MR (10 nM). Cells were washed and stained with a combination of directly conjugated antibodies (CD3-FITC, CD25-PE, CD3-Tricolor, CD3-FITC, HLA-DR-PE, CD3-Tricolor) and analyzed by FACS.

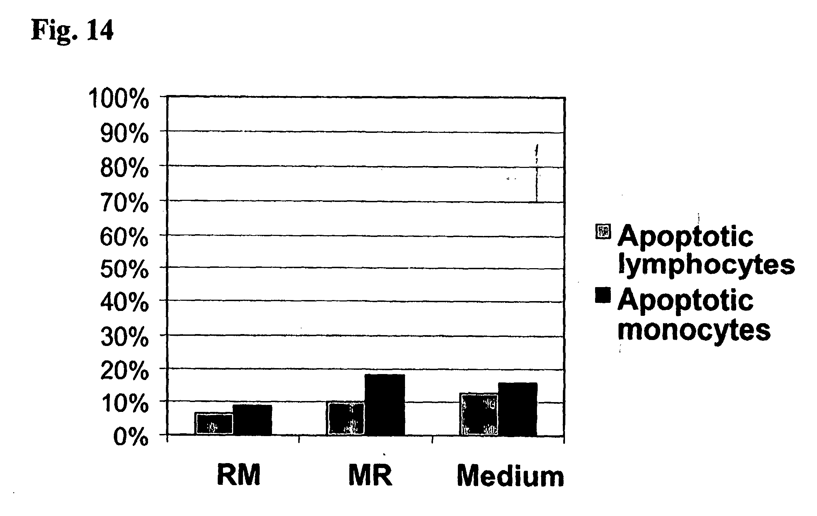

### Fig. 14: RM and MR do not induce apoptosis of human PBMC

PBMC were incubated for 24 h in medium alone or in medium containing RM (10 nM) or MR (10 nM). The cells were stained with Annexin V-FITC and propidium-iodide and analyzed by FACS. The number of apoptotic cells (Annexin V FITC positive and propidium iodide negative) was quantified by Cell-Quest analysis software.

The examples illustrate the invention:

### Example 1

A novel set of anti-CCR5 mAbs and their functional properties were determined. The ability of the mAbs to mediate receptor activation, as well as their influence on receptor trafficking were studied. MC-1 induced a dose-dependent downregulation of the cell surface CCR5 receptor on CHO cells (Fig. 1). In contrast to CHO cells MC-1 induced only a very weak endocytosis of CCR5 receptor on PBMC (not shown). In particular dimerization of CCR5 was necessary for CCR5 internalization with MC-1 antibody.

### Generation and epitope mapping of anti-CCR5 mAbs

Mice were immunized with CHO cells expressing human CCR5. Two CCR5-specific mAbs (MC-1 and MC-4) were isolated and further characterized (Mack et al. 1998). As shown by flow cytometry both mAbs bound CCR5 with high affinity. Both mAbs stained CCR5 on monocytic and lymphocytic populations of freshly isolated human PBMCs.

### MC-1.promotes receptor endocytosis through CCR5 dimerization

The ability of the monoclonal antibodies described herein above to induce CCR5 internalization in CHO cells was investigated by FACS analysis. 5 x 10⁵ CHO cells expressing CCR5 were incubated for 30 minutes at 37°C with chemokines or mAbs. Cells were then placed on ice, incubated with 15 µg MC-1 or MC-4 for 1 hour, washed with cold PBS, and stained with FITC-conjugated anti-mouse Ig Ab (F313, Dako). Cells were washed and analyzed on a FACSCalibur (Becton Dickinson). As shown in figure 1, MC-1 induced a dose-dependent downmodulation of the cell surface receptor. As measured by FACS analysis, 5 µg/ml MC-1 induced a 50% decrease of cell surface CCR5, similar to the level of downmodulation obtained with 100 nM RANTES. No significant endocytosis was seen when cells were incubated on ice with MC-1 (data not shown). Incubation of cells at 37°C with MC-4 had no effect (Fig. 1).

In order to test whether receptor endocytosis promoted by MC-1 is the consequence of MC-1 induced dimerization, the functional properties of a monovalent version of MC-1 were analyzed by flow cytometry. The light and heavy variable domains of MC-1 were cloned by PCR amplification using Pfu polymerase (Orlandi *et al.,* 1989). As described previously, the two domains were joined by a linker coding for (Gly4Ser)₃ (SEQ ID NO.: 54) and a C-terminal tail encoding 6 histidines was attached to facilitate purification. The single-chain fragment was expressed in the periplasmic space of E. coli and purified by Ni-NTA (Qiagen) as described (Mack et *al*., 1995). A single chain fragment of MC-1 (scFv-MC-1) tagged with poly-histidine continued to bind CCR5 with high affinity (data not shown). CCR5 internalization promoted by scFv-MC-1 was quantified by FACS analysis. CCR5 + CHO cells were incubated for 45 min with 3 µg/ml scFv-MC-1, on ice or 37°C as indicated. Cells were washed twice with cold PBS, and incubated with 4 µg/ml anti-His Ab (Dianova) for 45 min, on ice or at 37°C as indicated. Cells were washed again with cold PBS and stained with PE-labelled rabbit anti-mouse F(ab)₂ (Dako), and analyzed on a FACSCalibur using CellQuest software. CCR5 surface expression of cells incubated on ice with both scFv-MC-1 and anti-His mAb was used as control (100% fluorescence). As shown in Fig. 2, the incubation of cells with scFv-MC-1 at 37°C followed by anti-His antibody on ice did not significantly reduce surface expression of CCR5, indicating that monovalent MC-1 could not induce CCR5 downmodulation. However, when scFv-MC-1 and anti-His mAb were both incubated at 37°C, CCR5 surface fluorescence decreased by 50%, indicating that crosslinking of scFv-MC-1 can restore the effect of the parental divalent antibody. Taken together, these results demonstrate that receptor dimerization induced by MC-1 is necessary for CCR5 internalization.

### MC-4 inhibits CCR5 endocytosis.

It was investigated whether CCR5 specific antibodies could inhibit CCR5 internalization mediated by chemokines. Cells were preincubated with MC-4, then: with chemokines at 37°C, and surface expression of CCR5 was measured by flow cytometry. CHO cells expressing CCR5 were incubated on ice with medium or MC-4 for 20 min. Cells were then incubated with RANTES or AOP-RANTES for 30 min at 37°C. The cells were washed, incubated with 15 µg/ml MC-4 followed by FITC-conjugated anti-mouse Ig Ab (F313, Dako). As described, AOP-RANTES was more potent than RANTES in mediating CCR5 internalization (Fig. 3), inducing 50% or 90% receptor downmodulation at 1 and 100 nM, respectively. MC-4 inhibited RANTES (100 nM)-induced CCR5 endocytosis by more than 75% (Fig. 3). In the presence of MC-4 (1.5 µg/ml), a 10 fold higher concentration of AOP-RANTES was required to induce 50 % receptor downmodulation (Fig. 3). A stronger effect was found when higher MC-4 concentration was used.

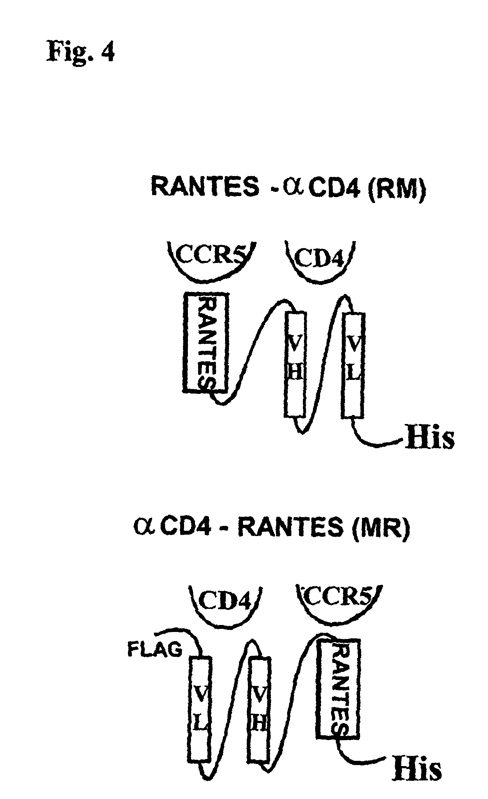

### Example 2: Construction of bifunctional chemokine constructs (Fig. 4)

RNA was isolated from the hybridoma MT-413 and reverse transcribed into cDNA using oligohexamers (Roche) and Superscript Reverse Transcriptase (Gibco). The VH domain of MT-413 was amplified by PCR with Pfu-polymerase (Stratagene) using the primer 1 (SEQ ID NO:31) and primer 2 (SEQ ID NO.:32). The PCR product was subcloned into the PCR-Script vector using the PCR-Script Amp cloning kit (Stratagene). Sequence 1 (SEQ ID NO.:33) was obtained. The VL domain from MT-413 was amplified by PCR with Pfu-polymerase (Stratagene) using the primer 3 (SEQ ID NO.: 34) and primer 4 (SEQ ID NO.:35). The PCR product was subcloned into the PCR-Script vector using the PCR-Script Amp cloning kit (Stratagene). Sequence 2 (SEQ ID No.:36) was obtained. A single-chain Fv fragment from the antibody MT-413 in the order VL-VH was generated by fusion PCR: for that purpose the VL domain (Sequence 2) (SEQ ID No.:36) was amplified with the primer 5 (SEQ ID NO:37) and primer 6 (SEQ ID NO:38) and the VH domain (Sequence 1) was amplified with the primer 7 (SEQ ID NO.:39) and primer 8 (SEQ ID NO.:40). Subsequently a fusion PCR reaction was performed with both PCR products and subcloned in frame with EcoRV and Sal1 into a periplasmic expression vector that already contained the OmpA signal sequence followed by the FLAG-Sequence and a EcoRV restriction site. Sequence 3 (SEQ ID NO.:41) was obtained. The MR construct was generated by fusion PCR. For that purpose the MT-413 VL-VH scFv was amplified with the primer 5 (SEQ ID NO:37) and primer 9 (SEQ ID NO.:42) and a RANTES cDNA (SEQ ID NO.:13) was amplified with the primer 10 (SEQ ID NO.:43) and primer 11 (SEQ ID NO:44). Subsequently a fusion PCR reaction was performed with both PCR products and subcloned in frame with EcoRV and Sal1 into a periplasmic expression vector that already contained the Omp-A signal sequence followed by the FLAG-sequence and a EcoRV restriction site. MR-construct (SEQ ID NO 19) was obtained and expressed in the periplasmatic space of E. coli.

A single-chain Fv fragment from the antibody MT-413 in the order VH-VL was generated by fusion PCR: for that purpose the VH domain (Sequence 1) (SEQ ID NO:33) was amplified with the primer 12 (SEQ ID NO.:45) and primer 9 (SEQ ID NO.:42) and the VL domain (Sequence 2) (SEQ ID No.:36) was amplified with the primer 13 (SEQ ID NO.:46) and primer 14 (SEQ ID NO.:47). Subsequently a fusion PCR reaction was performed with both PCR products and subcloned in frame with EcoRV and Sal1 into a periplasmic expression vector that already contained the OmpA signal sequence followed by the FLAG-sequence and a EcoRV restriction site.

For generation of the RM construct a PCR fragment was generated from RANTES cDNA (SEQ ID NO.: 13) with the primer 15 (SEQ ID NO.:48) and primer 16 (SEQ ID NO.:49). The PCR product was subcloned in frame with Fspl and BspE1 into a periplasmic expression vector that already contained a OmpA signal sequence with a Fspl restriction site. A PCR fragment generated from (MT-413 scFv VH-VL), see above with the primer 17 (SEQ ID NO.:50) and primer 14 (SEQ ID NO.:47:) was subcloned into this vector with BspE1 and Sal1 resulting in the RM construct (SEQ ID NO.: 17) and expressed in the periplasmatic space of E. coli.

These constructs were also produced in mammalian CHO cells leading to functional RM and MR molecules. Further cell lines are envisaged (CHO K1, HEK293, X63, SP2 cells, yeast).

These constructs were purified by affinity chromatography using the binding of His-Tag to immobilized 2⁺ metal ions such as Ni²⁺.

### List of primers:

nucleotides 1-59 = leader nucleotides 1-24 = flag-epitope

### Example 3: Binding of RM and MR to CCR5 as shown by ligand competition assay

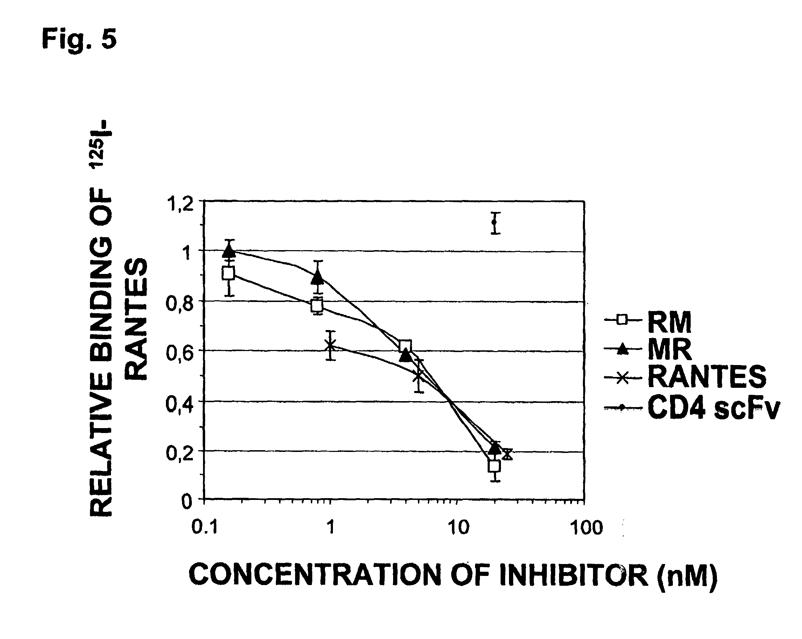

CHO cells stably transfected with CCR5 or CXCR4 were coincubated for 4 hours on ice with one of several inhibitors (RM, MR, RANTES or CD4 scFv) and 1 nM ¹²⁵I-RANTES. After washing with PBS cell bound radioactivity was counted with a gamma-counter. Cell bound radioactivity on CXCR4⁺ CHO cells was subtracted from cell bound radioactivity on CCR5⁺ CHO cells for each concentration of the inhibitor. Relative binding of ¹²⁵I-RANTES is calculated as (specific binding [inhibitor] / specific binding [medium]). All incubations were performed in triplicates. Error bars indicate the standard deviation (Fig. 5).

### Example 4: Specific binding of chemokine constructs RM and MR to CD4 on T cells

PBMC were incubated for 1 h on ice with the constructs RM and MR in various concentrations. After washing with PBS, cell bound constructs were detected with an antibody against 6xHis (Dianova) or an antibody against RANTES (VL-1) followed by incubation with PE-labelled rabbit-anti-mouse F(ab) fragments (Dako R439). Binding of RM and MR to CD4 on T cells was detected by FACS analysis (Fig. 6A). Concentration dependent binding of RM and MR to CD4+ T cells is shown in Fig. 6B.

### Example 5: Simultaneous binding of RM and MR to CCR5 and CD4 on human T cells

Human PBMC were incubated for 30 min at 37° C with various concentrations of RM (Fig. 7A), MR (Fig. 7B) and RANTES. Cells were then stained on ice for 1 h with a combination of directly conjugated antibodies against CCR5, CD8 and CD4 (CCR5-PE, Pharmingen, CD8-Cychrome, Immunotech and CD4-APC, Immunotech). CD4+ and CD8+ T cells were identified by expression of CD4 and CD8 as well as by their light scatter properties. Mean channel fluorescence (MCF) of CCR5 was quantified on CD4 and CD8 positive T cells by FACS-analysis. Relative surface expression of CCR5 was calculated as [MCF(exp.)-MCF(negative control)]/[(MCF(medium)-MCF(negative control).

### Example 6: Blocking of binding of anti-CD4 antibodies by chemokine constructs

PBMC were preincubated with various concentrations of RM, MR or RANTES for 1 h on ice. Without washing the cells CD4-FITC antibodies (Immunotech) were added in a dilution of 1:20 and incubated for 1 h on ice. Binding of CD4-FITC was detected by FACS analysis and the mean channel fluorescence (MCF) of CD4-FITC binding was quantified. Relative binding of CD4-FITC was calculated as [MCF(inhibitor)-MCF(negative control)]/[MCF(medium)-MCF(negative control) (Fig. 8A). Concentration dependent blockade of CD4 by RM and MR is shown in Fig. 8B.

### Example 7: Downmodulation of CCR5 expression on CHO cells induced by chemokine constructs

CHO cells expressing CCR5 or both CCR5 and CD4 were incubated for 30 min at 37°C with various concentrations of RANTES or RM (RANTES-anti-CD4) or medium as control Surface expression of CCR5 was measured by FACS analysis. For that purpose cells were stained with an antiserum against CCR5 generated in rabbits followed by FITC labelled goat anti rabbit antibody. As negative control cells were stained with preimmune serum instead of the antiserum. Surface CCR5 expressions was calculated as described (Mack M, Schloendorff, Methods Mol. Biol. 2000;138, 191-195). (Fig. 9)

### Example 8: Internalization of CCR5 on human PBMC induced by chemokine constructs

Human PBMC were cultured overnight to induce expression of CCR5 on monocytes. Cells were incubated for 30 min at 37° C with various concentrations of RM, MR and RANTES. Surface expression of CCR5 was then quantified by FACS-analysis (Fig. 10). For that purpose cells were stained on ice with the antibody MC-1 (5µg/ml) followed by PE-conjugated rabbit anti mouse F(ab)₂ fragments. After incubation for 10 min with mouse serum (10%) cells were stained with directly conjugated CD8-Cychrome and CD14-APC antibodies (Immunotech). Monocytes and CD8+ T cells were identified by expression of CD14 and CD8 as well as by their light scatter properties.

### Example 9: Internalization of CCR5 on human PBMC after washing out of chemokine constructs and RANTES

PBMC were preincubated for 30 min on ice with various concentrations of RANTES, RM and medium as control and then washed three times at 4°C. The cells were warmed up to 37°C and incubated for 30 min at 37°C. Surface expression of CCR5 was quantified on CD4+ and CD8+ T cells by FACS analysis. For that purpose cells were stained with the CCR5 antibody MC-1 (10 µg/ml) or IgG1 isotype control for 1 h on ice, followed by a FITC labelled mAb against mouse IgG1 for 1 h on ice. After washing, cells were stained with Phycoerythrin-Cyanin5 labelled CD8 and Allo-Phycocyanin labelled CD3 antibodies. Analysis was performed on a FACScalibur with CellQuest software (Fig. 11). Lymphocytes were gated by their forward and sideward light scatter properties. CD8 T cells were identified by expression of CD8 and CD3. CD4+ T cells were identified by expression of CD3 and absence of CD8. CCR5 surface expression was calculated as described (Mack M, Schloendorff D. Downmodulation and recycling of chemokine receptors. Methods Mol Biol. 2000;138:191-195).

### Example 10:Blocking of infection with M-tropic HIV-1 strains by RM construct, MR construct and a mixture of RANTES and anti-CD4scFv

PBMC were isolated from full blood of healthy donors by ficoll density centrifugation and cultured overnight with 20 U/ml IL-2. Cells were preincubated with inhibitors at concentrations of 20 nM (Fig. 12A) or 4 nM (Fig. 12B). After 3 h HIV-1 virus was added. Two M-tropic strains SF162 and BAL were used as well as one T-tropic strain IIIB. After incubation for 8 days RT-activity was measured with a commercial kit according to manufactures recommendations. All incubations were performed in quadruplicates and reproduced at least three times. Relative RT activity was calculated as described in Fig. 12. Error bars indicate the standard deviation.

The HIV infection blocking activity of the MR and RM constructs was compared with the HIV infection blocking activity of the mixture of RANTES and anti-CD4scFv (Fig. 12C). PBMC were isolated from full blood of healthy donors by ficoll density centrifugation and cultured overnight with 20 Ulml IL-2. Cells were preincubated with inhibitors at concentrations of 20 nM each. After 3 h the M-tropic HIV-1 virus SF 162 was added. After incubation for 9 days RT-activity was measured with a commercial kit according to manufactures recommendations. All incubations were performed in quadruplicates.

Surprisingly, the fusion proteins MR and RM were significantly more effective in blocking M-tropic HIV-1 infection than the combination of RANTES and anti-CD4 scFv. This demonstrates that the proximity of RANTES and anti-CD4scFv is important for the improvement of HIV blocking.

### Example 11: RM and MR constructs do not inhibit MLR

PBMC were obtained by Ficoll density centrifugation from the buffy coats of healthy donors. PBMC from two different donors were co-incubated for 14 days either in medium alone (RPMI 1640 with 10 % heat inactivated FCS and Pen/Strep) or in medium containing RM (10 nM) or MR (10 nM). Cells were recovered, washed once and stained with a combination of directly conjugated antibodies. These were either CD3-FITC (Becton-Dickinson), CD25-PE (Becton-Dickinson) and CD3-Tricolor (Caltag) or CD3-FITC (Becton-Dickinson), HLA-DR-PE (Becton-Dickinson) and CD3-Tricolor (Caltag). The cells were analyzed by flowcytometry using CellQuest analysis software (Fig. 13).

### Example 12: RM and MR do not induce apoptosis of human PBMC

PBMC were incubated for 24 h in medium alone (RPMI 1640 with 10 % heat inactivated FCS and Pen/Strep) or in medium containing RM (10 nM) or MR (10 nM). The cells were recovered and stained with Annexin V-FITC and propidium-iodide. Cells were analyzed by flowcytometry and the number of apoptotic cells (Annexin V FITC positive and propidium iodide negative) was quantified by Cell-Quest analysis software (Fig. 14).

### Example 13: An in vivo assay for analyzing the blocking of HIV-infection

For this illustrative assay, SCID (Severe Combined Immunodeficient) - mice are chosen that are suitable for engraftment of human hematolymphoid cells (e.g. NOD/LtSz-Rag1null or NOD/LtSz-scid mice described in Shultz et al. (2000) J Immunol. 164:2496-507). The mice are infected by i.p. injection with HI-virus 2-3 weeks after engraftment of human PBMC. A few hours before HIV- infection or after the infection the inhibitors RANTES-antiCD4 (RM), antiCD4-RANTES (MR), RANTES alone, anti-CD4 alone or a combination of RANTES and antiCD4 scFv are injected i.p. or i.v. in different time intervals (ranging from twice daily to every third day) and different amounts (ranging from 1 µg to 500 µg per mouse) until the end of the experiment. Three, four or eight weeks after HIV-1 infection, mice are killed and blood and spleen samples are collected. The samples are analyzed for percentages of human CD45+ cells by flow cytometry and for the presence of HIV-1 DNA and RNA by PCR. Aliquots of 200 µl of plasma is frozen at -80°C for subsequent quantitative HIV-1 RNA analysis using Amplicor RNA PCR kits (Roche Diagnostic Systems). Samples of 100 µl of whole blood and spleen cells are reprocessed for DNA and frozen at -80°C. These samples are later analyzed quantitatively (spleen) or qualitatively (blood) using the Amplicor PCR Assay system for DNA.

## Claims

1. A chemokine construct comprising a scFv anti-CD4 and a RANTES chemokine or a functional fragment thereof, whereby said chemokine construct has the structural domain form of
(a) RANTES-linker-(VH) anti-CD4-linker-(VL) anti-CD4; or
(b) (VL) anti-CD4-linker-(VH) anti-CD4 -linker-RANTES, or
(c) (VH) anti-CD4-linker (VL) anti-CD4 -linker-RANTES,
whereby in the alternatives (b) and (c), the linker between (VH) anti-CD4 and RANTES or between (VL) anti-CD4 and RANTES does not terminally comprise a methionine, a lysine or an isoleucine directly adjacent to the first amino acid of RANTES.

2. The chemokine construct of claim 1, wherein the scFv anti-CD4 is derived from a monoclonal anti-CD4 antibody.

3. The chemokine construct of claim 1 or 2, wherein said scFv anti-CD4 is derived from the monoclonal antibody MT413 comprising SEQ ID NO.:28.

4. The chemokine construct of any one of claims 1 to 3, wherein said (VH) anti-CD4 has at least one CDR3 consisting of the amino acid sequence: Lys Gly Glu Asn Gly Asn Ser Leu Ala Phe Ala Tyr (SEQ, ID No. 2).

5. The chemokine construct of any one of claims 1 to 4, wherein said (VH) anti-CD4 has at least one CDR2 consisting of the amino acid sequence : Glu Ile Tyr Pro Gly Ser Gly Ser Asp Tyr Tyr Asn Glu Asn Leu Lys Asp (SEQ ID No. 4).

6. The chemokine construct of any one of claims 1 to 4, wherein said (VH) anti-CD4 has at least one CDR1 consisting of the amino acid sequence : Asp Tyr Val Ile Asn (SEQ ID No. 6).

7. The chemokine construct of any one of claims 1 to 4, wherein the DNA sequence encoding said (VH) anti-CD4 has at least one CDR3 consisting of the nucleic acid sequence : aag ggg gag aat ggt aac tcc ctg gcg ttt gct tac (SEQ ID No. 1).

8. The chemokine construct of any one of claims 1 to 4, wherein the DNA sequence encoding said (VH) anti-CD4 has at least one CDR2 consisting of the nucleic acid sequence : gag att tat cct gga agt ggt agt gat tac tat aat gag aac etc aag gac (SEQ ID No. 3).

9. The chemokine construct of any one of claims 1 to 4, wherein the DNA sequence encoding said (VH) anti-CD4 has at least one CDR1 consisting of the nucleic acid sequence : gac tat gtt ata aat (SEQ ID No. 5).

10. The chemokine construct of any one of claims 1 to 4, wherein said (VL) anti-CD4 has at least one CDR3 consisting of the amino acid sequence : Gln Gln Ser Ile Gln Asp Pro Cys Thr (SEQ ID No. 8).

11. The chemokine construct of any one of claims 1 to 4, wherein said (VL) anti-CD4 has at least one CDR2 consisting of the amino acid sequence : Ala Ala Ser Asn Leu Glu Ser (SEQ ID No. 10).

12. The chemokine construct of any one of claims 1 to 4, wherein said (VL) anti-CD4 has at least one CDR1 consisting of the amino acid sequence : Gln Ser Val Asp Tyr Asp Gly Asp Ser Tyr Met Asn (SEQ ID No. 12).

13. The chemokine construct of any one of claims 1 to 4, wherein the DNA sequence encoding said (VL) anti-CD4 has at least one CDR3 consisting of the nucleic acid sequence : cag caa agt att cag gat ccg tgc acg (SEQ ID No. 7).

14. The chemokine construct of any one of claims 1 to 4, wherein the DNA sequence encoding said (VL) anti-CD4 has at least one CDR2 consisting of the nucleic acid sequence : gct gca tcc aat cta gaa tct (SEQ ID No. 9).

15. The chemokine construct of any one of claims 1 to 4, wherein the DNA sequence encoding said (VL) anti-CD4 has at least one CDR1 consisting of the nucleic acid sequence : caa agt gtt gat tat gat ggt gat agt tat atg aac (SEQ ID No. 11).

16. The chemokine construct of any one of claims 1 to 15, wherein said RANTES or RANTES fragments is a RANTES
(a) comprising an amino acid sequence
(b) which is encoded by a DNA comprising the nucleic acid sequence the nucleic acid sequence the nucleic acid sequence: the nucleic acid sequence

17. The chemokine construct of any one of claims 1 to 16, wherein said linker between (VH) anti-CD4 or (VL) anti-CD4 and RANTES comprises between 3 and 30 amino acids.

18. The chemokine construct of claim 17, wherein said linker comprises between 5 and 15 amino acids.

19. The chemokine construct of any one of claims 1 to 16, wherein said linker between (VH) anti-CD4 and (VL) anti-CD4 comprises between 8 and 20 amino acids.

20. The chemokine construct of claim 19, wherein said linker comprises between 13 and 17 amino acids.

21. The chemokine construct of any one of claims 17 and 20, wherein said linker comprises the amino acid sequence Gly Gly Gly Gly Ser (SEQ ID NO.:51).

22. The chemokine construct of any one of claims 1 to 21, wherein said chemokine construct further comprises a tag.

23. The chemokine construct of claim 22, wherein said tag is a FLAG-tag or a HIS-tag.

24. The chemokine construct of any one of claims 1 to 23, wherein said RANTES-linker-(VH)anti-CD4-linker-(VL)anti-CD4 construct comprises an amino acid sequence wherein said (VL)anti-CD4-linker-(VH)anti-CD4-linker RANTES construct comprises an amino acid sequence or
wherein said (VL)anti-CD4-linker-(VH)anti-CD4-linker-RANTES construct comprises an amino acid sequence or wherein said RANTES-linker-(VH)anti-CD4-linker-(VL)anti-CD4 construct is encoded by a nucleic acid sequence wherein said (VL)anti-CD4-linker-(VH)anti-CD4-linker-RANTES construct is encoded by a nucleic acid sequence wherein said (VL)anti-CD4-finker-(VH)anti-CD4-linker-RANTES construct is encoded by a nucleic acid sequence

25. The chemokine construct of any one of claims 1 to 24, wherein said chemokine construct further comprises an additional cytokine or chemokine linked to the scFv anti-CD4 part.

26. The chemokine construct of claim 25, wherein said chemokine is (SDF-1) stromal- cell derived factor 1.

27. The chemokine construct of claim 26, wherein said construct has the structural domain form of
(a) RANTES-linker-(VH) anti-CD4-linker-(VL) anti-CD4-linker-SDF-1; or
(b) SDF1-linker-(VL) anti-CD4-linker-(VH) anti-CD4 -linker-RANTES, or
(c) SDF1-linker- (VH) anti-CD4-linker-(VL) anti-CD4 -linker-RANTES.

28. A polynucleotide which upon expression encodes a chemokine construct of any one of claims 1 to 27.

29. The polynucleotide of claim 28, wherein said polynucleotide is
(a) a polynucleotide comprising the nucleic acid molecule in particular encoding the polypeptide as depicted in SEQ ID NO: 18, SEQ ID NO: 20 or SEQ ID NO: 30;
(b) a polynucleotide comprising the nucleic acid molecule as depicted in SEQ ID NO: 17, SEQ ID NO: 19 or SEQ ID NO: 29; or
(c) a polynucleotide hybridizing under stringent conditions to the complementary strand of a polynucleotide of (a) or (b).

30. A vector comprising the polynucleotide of claim 28.

31. A cell transfected with the polynucleotide of claim 28 or the vector of claim 30.

32. A method for the preparation of the chemokine construct of any one of claims 1 to 27 comprising the cultivation of a cell of claim 31 and isolating said construct from the culture.

33. A composition comprising the chemokine construct of any one of claims 1 to 27, the polynucleotide of claim 28 or the vector of claim 30.

34. The composition of claim 33 which is a pharmaceutical composition optionally further comprising, a pharmaceutically acceptable carrier.

35. Use of the polynucleotide of claim 28 or the vector of claim 30 for the preparation of compositions for gene therapy.

36. Use of a chemokine construct of any one of claims 1 to 27, a polynucleotide of claim 28 or a vector of claim 30 for the preparation of a pharmaceutical composition for the treatment of a HIV-infection or of AIDS.

37. Use of a chemokine construct of any one of claims 1 to 27, a polynucleotide of claim 28 or a vector of claim 30 for the preparation of a pharmaceutical composition for the treatment of inflammatory diseases and/or autoimmune diseases.

## Patentansprüche

1. Chemokinkonstrukt, umfassend ein Anti-CD4-scFv und ein RANTES-Chemokin oder ein funktionelles Fragment davon, wobei das Chemokinkonstrukt die folgende Strukturdomänenform besitzt:
(a) RANTES-Linker-(VH) Anti-CD4-Linker-(VL) Anti-CD4; oder
(b) (VL) Anti-CD4-Linker-(VH) Anti-CD4-Linker-RANTES, oder
(c) (VH) Anti-CD4-Linker-(VL) Anti-CD4-Linker-RANTES,
wobei in den Alternativen (b) und (c) der Linker zwischen (VH) Anti-CD4 und RANTES oder zwischen (VL) Anti-CD4 und RANTES nicht an einem Ende ein Methionin, ein Lysin oder ein Isoleucin direkt angrenzend an die erste Aminosäure von RANTES umfasst.

2. Chemokinkonstrukt nach Anspruch 1, wobei das Anti-CD4-scFv abgeleitet ist von einem monclonalen Anti-CD4-Antikörper.

3. Chemokinkonstrukt nach Anspruch 1 oder 2, wobei der Anti-CD4-scFv abgeleitet ist von einem monoclonalen Antikörper MT413, umfassend SEQ ID NR:28.

4. Chemokinkonstrukt nach einem der Ansprüche 1 bis 3, wobei die (VH) Anti-CD4 mindestens eine CDR3 besitzt, bestehend aus der Aminosäuresequenz: Lys Gly Glu Asn Gly Asn Ser Leu Ala Phe Ala Tyr (SEQ ID NR:2).

5. Chemokinkonstrukt nach einem der Ansprüche 1 bis 4, wobei die (VH) Anti-CD4 mindestens eine CDR2 besitzt, bestehend aus der Aminosäuresequenz: Glu Ile Tyr Pro Gly Ser Gly Ser Asp Tyr Tyr Asn Glu Asn Leu Lys Asp (SEQ ID NR:4).

6. Chemokinkonstrukt nach einem der Ansprüche 1 bis 4, wobei die (VH) Anti-CD4 mindestens eine CDR1 besitzt, bestehend aus der Aminosäuresequenz: Asp Tyr Val Ile Asn (SEQ ID NR:6).

7. Chemokinkonstrukt nach einem der Ansprüche 1 bis 4, wobei die DNA-Sequenz, die (VH) Anti-CD4 codiert, mindestens eine CDR3 besitzt, bestehend aus der Nucleinsäuresequenz: aag ggg gag aat ggt aac tcc ctg gcg ttt gct tac (SEQ ID NR:1).

8. Chemokinkonstrukt nach einem der Ansprüche 1 bis 4, wobei die DNA-Sequenz, die (VH) Anti-CD4 codiert, mindestens eine CDR2 besitzt, bestehend aus der Nucleinsäuresequenz: gag att tat cct gga agt ggt agt gat tac tat aat gag aac ctc aag gac (SEQ ID NR:3).

9. Chemokinkonstrukt nach einem der Ansprüche 1 bis 4, wobei die DNA-Sequenz, die (VH) Anti-CD4 codiert, mindestens eine CDR1 besitzt, bestehend aus der Nucleinsäuresequenz: gac tat gtt ata aat (SEQ ID NR:5).

10. Chemokinkonstrukt nach einem der Ansprüche 1 bis 4, wobei die (VL) Anti-CD4 mindestens eine CDR3 besitzt, bestehend aus der Aminosäuresequenz: Gln Gln Ser Ile Gln Asp Pro Cys Thr (SEQ ID NR:8).

11. Chemokinkonstrukt nach einem der Ansprüche 1 bis 4, wobei die (VL) Anti-CD4 mindestens eine CDR2 besitzt, bestehend aus der Aminosäuresequenz: Ala Ala Ser Asn Leu Glu Ser (SEQ ID NR:10).

12. Chemokinkonstrukt nach einem der Ansprüche 1 bis 4, wobei die (VL) Anti-CD4 mindestens eine CDR1 besitzt, bestehend aus der Aminosäuresequenz: Gln Ser Val Asp Tyr Asp Gly Asp Ser Tyr Met Asn (SEQ ID NR:12).

13. Chemokinkonstrukt nach einem der Ansprüche 1 bis 4, wobei die DNA-Sequenz, die (VL) Anti-CD4 codiert, mindestens eine CDR3 besitzt, bestehend aus der Nucleinsäuresequenz: cag caa agt att cag gat ccg tgc acg (SEQ ID NR: 7).

14. Chemokinkonstrukt nach einem der Ansprüche 1 bis 4, wobei die DNA-Sequenz, die (VL) Anti-CD4 codiert, mindestens eine CDR2 besitzt, bestehend aus der Nucleinsäuresequenz: gct gca tcc aat cta gaa tct (SEQ ID NR:9).

15. Chemokinkonstrukt nach einem der Ansprüche 1 bis 4, wobei die DNA-Sequenz, die (VL) Anti-CD4 codiert, mindestens eine CDR1 besitzt, bestehend aus der Nucleinsäuresequenz: caa agt gtt gat tat gat ggt gat agt tat atg aac (SEQ ID NR: 11).

16. Chemokinkonstrukt nach einem der Ansprüche 1 bis 15, wobei das RANTES oder RANTES-Fragmente ein RANTES ist,
(a) umfassend eine Aminosäuresequenz eine Aminosäuresequenz eine Aminosäuresequenz eine Aminosäuresequenz oder
(b) welches codiert wird durch eine DNA, umfassend die Nucleinsäuresequenz die Nucleinsäuresequenz die Nucleinsäuresequenz: oder
die Nucleinsäuresequenz

17. Chemokinkonstrukt nach einem der Ansprüche 1 bis 16, wobei der Linker zwischen (VH) Anti-CD4 oder (VL) Anti-CD4 und RANTES zwischen 3 und 30 Aminosäuren umfasst.

18. Chemokinkonstrukt nach Anspruch 17, wobei der Linker zwischen 5 und 15 Aminosäuren umfasst.

19. Chemokinkonstrukt nach einem der Ansprüche 1 bis 16, wobei der Linker zwischen (VH) Anti-CD4 und (VL) Anti-CD4 zwischen 8 und 20 Aminosäuren umfasst.

20. Chemokinkonstrukt nach Anspruch 19, wobei der Linker zwischen 13 und 17 Aminosäuren umfasst.

21. Chemokinkonstrukt nach einem der Ansprüche 17 und 20, wobei der Linker die Aminosäuresequenz Gly Gly Gly Gly Ser (SEQ ID NR:51) umfasst.

22. Chemokinkonstrukt nach einem der Ansprüche 1 bis 21, wobei das Chemokinkonstrukt darüber hinaus eine Markierung umfasst.

23. Chemokinkonstrukt nach Anspruch 22, wobei die Markierung eine FLAG-Markierung oder eine HIS-Markierung ist.

24. Chemokinkonstrukt nach einem der Ansprüche 1 bis 23, wobei das RANTES-Linker-(VH) Anti-CD4-Linker-(VL) Anti-CD4-Konstrukt eine Aminosäuresequenz umfasst wobei das (VL) Anti-CD4-Lmker-(VH) Anti-CD4-Linker-RANTES-Konstrukt eine Aminosäuresequenz umfasst oder
wobei das (VL) Anti-CD4-Linker-(VH) Anti-CD4-Linker-RANTES-Konstrukt eine Aminosäuresequenz umfasst oder wobei das RANTES-Linker-(VH) Anti-CD4-Linker-(VL) Anti-CD4-Konstrukt codiert wird durch eine Nucleinsäuresequenz oder
wobei das (VL) Anti-CD4-Linker-(VH) Anti-CD4-Linker-RANTES-Konstrukt codiert wird durch eine Nucleinsäuresequenz oder
wobei das (VL) Anti-CD4-Linker-(VH) Anti-CD4-Linker-RANTES-Konstrukt codiert ist durch eine Nucleinsäuresequenz

25. Chemokinkonstrukt nach einem der Ansprüche 1 bis 24, wobei das Chemokinkonstrukt darüber hinaus ein weiteres Cytokin oder Chemokin umfasst, verbunden mit dem Anti-CD4-scFv-Teil.

26. Chemokinkonstrukt nach Anspruch 25, wobei das Chemokin Stromal-cell derived factor 1 (SDF-1) ist.

27. Chemokinkonstrukt nach Anspruch 26, wobei das Konstrukt folgende Strukturdomänenform besitzt
(a) RANTES-Linker-(VH) Anti-CD4-Linker-(VL) Anti-CD4-Linker-SDF-1; oder
(b) SDF-1-Linker-(VL) Anti-CD4-Linker-(VH) Anti-CD4-Linker-RANTES, oder
(c) SDF-1-Linker-(VH) Anti-CD4-Linker-(VL) Anb-CD4-Linker RANTES.

28. Polynucleotid, welches bei Expression ein Chemokinkonstrukt nach einem der Ansprüche 1 bis 27 codiert.

29. Polynucleotid nach Anspruch 28, wobei das Polynucleotid
(a) ein Polynucleotid ist, umfassend das Nucleinsäuremolekül, welches insbesondere das Polypeptid wie dargestellt in SEQ ID NR:18, SEQ ID NR:20 oder SEQ ID NR:30 codiert;
(b) ein Polynucleotid ist, umfassend das Nucleinsäuremoiekül wie dargestellt in SEQ ID NR:17, SEQ ID NR:19 oder SEQ ID NR:29; oder
(c) ein Polynucleotid ist, welches unter stringenten Bedingungen mit dem komplementären Strang eines Polynucleotids nach (a) oder (b) hybridisiert.

30. Vektor umfassend das Polynucleotid nach Anspruch 28.

31. Zelle, transfiziert mit dem Polynucleotid nach Anspruch 28 oder dem Vektor nach Anspruch 30.

32. Verfahren zur Herstellung des Chemokinkonstrukts nach einem der Ansprüche 1 bis 27, umfassend das Züchten einer Zelle nach Anspruch 31 und das Isolieren des Konstruktes aus der Kultur.

33. Zusammensetzung, umfassend das Chemokinkonstrukt nach einem der Ansprüche 1 bis 27, das Polynucleotid nach Anspruch 28 oder den Vektor nach Anspruch 30.

34. Zusammensetzung nach Anspruch 33, welche ein Arzneimittel ist, gegebenenfalls darüber hinaus umfassend einen pharmazeutisch verträglichen Träger.

35. Verwendung des Polynucleotids nach Anspruch 28 oder des Vektors nach Anspruch 30 zur Herstellung von Zusammensetzungen zur Gentherapie.

36. Verwendung eines Chemokinkonstrukts nach einem der Ansprüche 1 bis 27, eines Polynucleotids nach Anspruch 28 oder eines Vektors nach Anspruch 30 für die Herstellung eines Arzneimittels zur Behandlung einer HIV-Infektion oder von AIDS.

37. Verwendung eines Chemokinkonstrukts nach einem der Ansprüche 1 bis 27, eines Polynucleotids nach Anspruch 28 oder eines Vektors nach Anspruch 30 für die Herstellung eines Arzneimittels zur Behandlung von entzündlichen Erkrankungen und/oder Autoimmunerkrankungen.

## Revendications

1. Construit de chimiokine comprenant un scFv anti-CD4 et une chimiokine RANTES ou un fragment fonctionnel de celle-ci, dans lequel ledit construit de chimiokine présente la forme de domaine structural suivante :
(a) RANTES - lieur - (VH) anti-CD4 - lieur - anti-CD4 (VL) ; ou
(b) (VL) anti-CD4 - lieur - (VH) anti-CD4 - lieur -RANTES, ou
(c) (VH) anti-CD4 - lieur - (VL) anti-CD4 - lieur -RANTES,
dans lequel, dans les alternatives (b) et (c), le lieur entre (VH) anti-CD4 et RANTES ou entre (VL) anti-CD4 et RANTES ne comprend pas, en position terminale, une méthionine, une lysine ou une isoleucine directement adjacente au premier acide aminé de RANTES.

2. Construit de chimiokine selon la revendication 1, dans lequel le scFv anti-CD4 provient d'un anticorps monoclonal anti-CD4.

3. Construit de chimiokine selon la revendication 1 ou 2, dans lequel ledit scFv anti-CD4 provient de l'anticorps monoclonal MT413 comprenant SEQ ID NO :28.

4. Construit de chimiokine selon l'une quelconque des revendications 1 à 3, dans lequel ledit (VH) anti-CD4 a au moins un CDR3 constitué de la séquence d'acides aminés : Lys Gly Glu Asn Gly Asn Ser Leu Ala Phe Ala Tyr (SEQ ID No. 2).

5. Construit de chimiokine selon l'une quelconque des revendications 1 à 4, dans lequel ledit (VH) anti-CD4 a au moins un CDR2 constitué de la séquence d'acides aminés : Glu Ile Tyr Pro Gly Ser Gly Ser Asp Tyr Tyr Asn Glu Asn Leu Lys Asp (SEQ ID No.4).

6. Construit de chimiokine selon l'une quelconque des revendications 1 à 4, dans lequel ledit (VH) anti-CD4 a au moins un CDR1 constitué de la séquence d'acides aminés : Asp Tyr Val Ile Asn (SEQ ID No.6).

7. Construit de chimiokine selon l'une quelconque des revendications 1 à 4, dans lequel la séquence d'ADN codant ledit (VH) anti-CD4 a au moins un CDR3 constitué de la séquence d'acide nucléique : aag ggg gag aat ggt aac tcc ctg gcg ttt gct tac (SEQ ID No. 1).

8. Construit de chimiokine selon l'une quelconque des revendications 1 à 4, dans lequel la séquence d'ADN codant ledit (VH) anti-CD4 a au moins un CDR2 constitué de la séquence d'acide nucléique : gag att tat cct gga agt ggt agt gat tac tat aat gag aac ctc aag gac (SEQ ID No. 3).

9. Construit de chimiokine selon l'une quelconque des revendications 1 à 4, dans lequel la séquence d'ADN codant ledit (VH) anti-CD4 a au moins un CDR1 constitué de la séquence d'acide nucléique : gac tat gtt ata aat (SEQ ID No. 5).

10. Construit de chimiokine selon l'une quelconque des revendications 1 à 4, dans lequel ledit (VL) anti-CD4 a au moins un CDR3 constitué de la séquence d'acides aminés : Gln Gln Ser Ile Gln Asp Pro Cys Thr (SEQ ID No. 8).

11. Construit de chimiokine selon l'une quelconque des revendications 1 à 4, dans lequel ledit (VL) anti-CD4 a au moins un CDR2 constitué de la séquence d'acides aminés : Ala Ala Ser Asn Leu Glu Ser (SEQ ID No. 10).

12. Construit de chimiokine selon l'une quelconque des revendications 1 à 4, dans lequel ledit (VL) anti-CD4 a au moins un CDR1 constitué de la séquence d'acides aminés : Gln Ser Val Asp Tyr Asp Gly Asp Ser Tyr Met Asn (SEQ ID No. 12).

13. Construit de chimiokine selon l'une quelconque des revendications 1 à 4, dans lequel la séquence d'ADN codant ledit (VL) anti-CD4 a au moins un CDR3 constitué de la séquence d'acide nucléique : cag caa agt att cag gat ccg tgc acg (SEQ ID No. 7).

14. Construit de chimiokine selon l'une quelconque des revendications 1 à 4, dans lequel la séquence d'ADN codant ledit (VL) anti-CD4 a au moins un CDR2 constitué de la séquence d'acide nucléique : gct gca tcc aat cta gaa tct (SEQ ID No. 9).

15. Construit de chimiokine selon l'une quelconque des revendications 1 à 4, dans lequel la séquence d'ADN codant ledit (VL) anti-CD4 a au moins un CDR1 constitué de la séquence d'acide nucléique : caa agt gtt gat tat gat ggt gat agt tat atg aac (SEQ ID No. 11).

16. Construit de chimiokine selon l'une quelconque des revendications 1 à 15, dans lequel ledit RANTES ou fragments de RANTES est un RANTES
(a) comprenant une séquence d'acides aminés , une séquence d'acides aminés , une séquence d'acides aminés ou
(b) qui est codé par un ADN comprenant la séquence d'acide nucléique la séquence d'acide nucléique la séquence d'acide nucléique ou
la séquence d'acide nucléique

17. Construit de chimiokine selon l'une quelconque des revendications 1 à 16, dans lequel ledit lieur entre le (VH) anti-CD4 ou le (VL) anti-CD4 et RANTES comprend entre 3 et 30 acides aminés.

18. Construit de chimiokine selon la revendication 17, dans lequel ledit lieur comprend entre 5 et 15 acides aminés.

19. Construit de chimiokine selon l'une quelconque des revendications 1 à 16, dans lequel ledit lieur entre le (VH) anti-CD4 et le (VL) anti-CD4 comprend entre 8 et 20 acides aminés.

20. Construit de chimiokine selon la revendication 19, dans lequel ledit lieur comprend entre 13 et 17 acides aminés.

21. Construit de chimiokine selon l'une quelconque des revendications 17 et 20, dans lequel ledit lieur comprend la séquence d'acides aminés Gly Gly Gly Gly Ser (SEQ ID NO. :51).

22. Construit de chimiokine selon l'une quelconque des revendications 1 à 21, dans lequel ledit construit de chimiokine comprend en outre une étiquette.

23. Construit de chimiokine selon la revendication 22, dans lequel ladite étiquette est une étiquette FLAG ou une étiquette HIS.

24. Construit de chimiokine selon l'une quelconque des revendications 1 à 23, dans lequel ledit construit RANTES-lieur-(VH) anti-CD4-lieur-(VL) anti-CD4 comprend une séquence d'acides aminés dans lequel ledit construit (VL) anti-CD4-lieur-(VH) anti-CD4-lieur-RANTES comprend une séquence d'acides aminés ou
dans lequel ledit construit (VL) anti-CD4-lieur-(VH) anti-CD4-lieur-RANTES comprend une séquence d'acides aminés ou dans lequel ledit construit RANTES-lieur-(VH) anti-CD4-lieur-(VL) anti-CD4 est codé par une séquence d'acide nucléique ou
dans lequel ledit construit (VL) anti-CD4-lieur-(VH) anti-CD4-lieur-RANTES est codé par une séquence d'acide nucléique ou
dans lequel ledit construit (VL) anti-CD4-lieur-(VH) anti-CD4-lieur-RANTES est codé par une séquence d'acide nucléique

25. Construit de chimiokine selon l'une quelconque des revendications 1 à 24, dans lequel ledit construit de chimiokine comprend en outre une cytokine ou une chimiokine additionnelle liée à la partie scFv anti-CD4.

26. Construit de chimiokine selon la revendication 25, dans lequel ladite chimiokine est le Facteur 1 Dérivé de cellules du Stroma (SDF-1).

27. Construit de chimiokine selon la revendication 26, dans lequel ledit construit présente la forme de domaine structural suivante:
(a) RANTES-lieur- (VH) anti-CD4 -lieur- anti-CD4 (VL)-lieur-SDF-1 ; ou
(b) SDF-1-lieur- (VL) anti-CD4 -lieur- (VH) anti-CD4 -lieur-RANTES, ou
(c) SDF-1-lieur- (VH) anti-CD4 -lieur- (VL) anti-CD4 -lieur-RANTES.

28. Polynucléotide qui lorsqu'il s'exprime code un construit de chimiokine selon l'une quelconque des revendications 1 à 27.

29. Polynucléotide selon la revendication 28, dans lequel ledit polynucléotide est
(a) un polynucléotide comprenant la molécule d'acide nucléique codant en particulier le polypeptide tel que décrit dans SEQ ID NO : 18, SEQ ID NO : 20 ou SEQ ID NO : 30 ;
(b) un polynucléotide comprenant la molécule d'acide nucléique telle que décrite dans SEQ ID NO : 17, SEQ ID NO : 19 ou SEQ ID NO : 29 ; ou
(c) un polynucléotide s'hybridant dans des conditions stringentes au brin complémentaire d'un polynucléotide selon (a) ou (b).

30. Vecteur comprenant le polynucléotide selon la revendication 28.

31. Cellule transfectée avec le polynucléotide selon la revendication 28 ou le vecteur selon la revendication 30.

32. Méthode de préparation d'un construit de chimiokine selon l'une quelconque des revendications 1 à 27 comprenant la culture d'une cellule selon la revendication 31 et l'isolement dudit construit à partir de la culture.

33. Composition comprenant le construit de chimiokine selon l'une quelconque des revendications 1 à 27, le polynucléotide selon la revendication 28 ou le vecteur selon la revendication 30.

34. Composition selon la revendication 33 qui est une composition pharmaceutique comprenant éventuellement en outre un support acceptable sur le plan pharmaceutique.

35. Utilisation du polynucléotide selon la revendication 28 ou du vecteur selon la revendication 30 pour la préparation de compositions pour la thérapie génique.

36. Utilisation d'un construit de chimiokine selon l'une quelconque des revendications 1 à 27, d'un polynucléotide selon la revendication 28 ou d'un vecteur selon la revendication 30 pour la préparation d'une composition pharmaceutique pour le traitement d'une infection par VIH ou du SIDA.

37. Utilisation d'un construit de chimiokine selon l'une quelconque des revendications 1 à 27, d'un polynucléotide selon la revendication 28 ou d'un vecteur selon la revendication 30 pour la préparation d'une composition pharmaceutique pour le traitement de maladies inflammatoires et/ou auto-immunes.


### SEQUENCE LISTING

<110> MACK, Matthias
<110> SCHLOENDORFF, Detlef
<120> Mono- and Dual Chemokine/Cytokine Constructs
<130> F3135 PCT
<140> To be assigned
   <141> 2002-12-21
<150> EP 01130746.9
   <151> 2001-12-21
<160> 54
<170> PatentIn version 3.1
<210> 1
   <211> 36
   <212> DNA
   <213> Mus musculus
<400> 1
   aaggggagaa atggtaactc cctggcgttt gcttac                                36
<210> 2
   <211> 12
   <212> PRT
   <213> Mus musculus
<400> 2
   Lys Gly Glu Asn Gly Asn Ser Leu Ala Phe Ala Tyr
   1               5                   10
<210> 3
   <211> 51
   <212> DNA
   <213> Mus musculus
<400> 3
   gagatttatc ctggaagtgg tagtgattac tataatgaga acctcaagga c 51
<210> 4
   <211> 17
   <212> PRT
   <213> Mus musculus
<400> 4
   Glu Ile Tyr Pro Gly Ser Gly Ser Asp Tyr Tyr Asn Glu Asn Leu Lys
   1 5 10 15
   Asp
<210> 5
   <211> 15
   <212> DNA
   <213> Mus musculus
<400> 5
   gactatgtta taaat 15
<210> 6
   <211> 5
   <212> PRT
   <213> Mus musculus
<400> 6
   Asp Tyr Val Ile Asn
   1 5
<210> 7
   <211> 27
   <212> DNA
   <213> Mus musculus
<400> 7
   cagcaaagta ttcaggatcc gtgcacg 27
<210> 8
   <211> 9
   <212> PRT
   <213> Mus musculus
<400> 8
   Gln Gln Ser Ile Gln Asp Pro Cys Thr
   1 5
<210> 9
   <211> 21
   <212> DNA
   <213> Mus musculus
<400> 9
   gctgcatcca atctagaatc t 21
<210> 10
   <211> 7
   <212> PRT
   <213> Mus musculus
<400> 10
   Ala Ala Ser Asn Leu Glu Ser
   1 5
<210> 11
   <211> 36
   <212> DNA
   <213> Mus musculus
<400> 11
   caaagtgttg attatgatgg tgatagttat atgaac 36
<210> 12
   <211> 12
   <212> PRT
   <213> Mus musculus
<400> 12
   Gln Ser Val Asp Tyr Asp Gly Asp Ser Tyr Met Asn
   1 5 10
<210> 13
   <211> 204
   <212> DNA
   <213> Homo sapiens
<400> 13
   tccccatatt cctcggacac cacaccctgc tgctttgcct acattgcccg cccactgccc      60
   cgtgcccaca tcaaggagta tttctacacc agtggcaagt gctccaaccc agcagtcgtc     120
   tttgtcaccc gaaagaaccg ccaagtgtgt gccaacccag agaagaaatg ggttcgggag     180
   tacatcaact ctttggagat gagc                                            204
<210> 14
   <211> 68
   <212> PRT
   <213> Homo sapiens
<400> 14
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
   Val Cys Ala Asn Pro Glu Lys Lys Trp Val Arg Glu Tyr Ile Asn Ser
       50                  55                  60
   Leu Glu Met Ser
   65
<210> 15
   <211> 204
   <212> DNA
   <213> Artificial Sequence
<220>
   <223> RANTES Glu66Gln nucleotide sequence
<400> 15
   tccccatatt cctcggacac cacaccctgc tgctttgcct acattgcccg cccactgccc      60
   cgtgcccaca tcaaggagta tttctacacc agtggcaagt gctccaaccc agcagtcgtc     120
   tttgtcaccc gaaagaaccg ccaagtgtgt gccaacccag agaagaaatg ggttcgggag     180
   tacatcaact ctttgcagat gagc                                            204
<210> 16
   <211> 68
   <212> PRT
   <213> Artificial Sequence
<220>
   <223> RANTES Glu66Gln
<400> 16
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Gln Met Ser
   65
<210> 17
   <211> 993
   <212> DNA
   <213> Artificial Sequence
<220>
   <223> RM construct
<400> 17
   tccccatatt cctcggacac cacaccctgc tgctttgcct acattgcccg cccactgccc      60
   cgtgcccaca tcaaggagta tttctacacc agtggcaagt gctccaaccc agcagtcgtc     120
   tttgtcaccc gaaagaaccg ccaagtgtgt gccaacccag agaagaaatg ggttcgggag     180
   tacatcaact ctttgcagat gagctcaggt ggaggtggct ccggaggtgg tggatccgat     240
   atccagctgc agcagtctgg acctgagctg gtgaagcctg gggcttcagt gaagatgtcc     300
   tgcaaggctt ctggatacac attcactgac tatgttataa attgggtgaa gcagagaact     360
   ggacagggcc ttgagtggat tggagagatt tatcctggaa gtggtagtga ttactataat     420
   gagaacctca aggacaaggc cacactgact gcagacaaat cctccaacac agcctacatg     480
   cagctcagta gcctgacatc tgaggactct gcggtctatt tctgtgcaag aaagggggag     540
   aatggtaact ccctggcgtt tgcttactgg ggccaaggga ccctggtcac cgtctccgca     600
   ggtggtggtg gttctggcgg cggcggctcc ggtggtggtg gtgacatcgt gctgacccag     660
   tctccagctt ctttggctgt gtctctaggg cagagggcca ccatctcctg caaggccagc     720
   caaagtgttg attatgatgg tgatagttat atgaactggt accaacagaa atcaggacag     780
   ccacccaaac tcctcatcta tgctgcatcc aatctagaat ctgggatccc agccaggttt     840
   agtggcagtg ggtctgggac agacttcacc ctcaacatcc atcctgtgga ggaggaggat     900
   gctgcgacct attactgtca gcaaagtatt caggatccgt gcacgttcgg aggggggacc    960
   gagctcgaga tcaaacatca tcatcaccat cat                                  993
<210> 18
   <211> 331
   <212> PRT
   <213> Artificial Sequence
<220>
   <223> RM construct
<400> 18
   Ser Pro Tyr Ser Ser Asp Thr Thr Pro Cys Cys Phe Ala Tyr Ile Ala
   1               5                   10                  15
   Arg Pro Leu Pro Arg Ala His Ile Lys Glu Tyr Phe Tyr Thr Ser Gly
               20                  25                  30
   Lys Cys Ser Asn Pro Ala Val Val Phe Val Thr Arg Lys Asn Arg Gln
           35                  40                  45
   Val Cys Ala Asn Pro Glu Lys Lys Trp Val Arg Glu Tyr Ile Asn Ser
       50                  55                  60
   Leu Gln Met Ser Ser Gly Gly Gly Gly Ser Gly Gly Gly Gly Ser Asp
   65                  70                  75                  80
   Ile Gln Leu Gln Gln Ser Gly Pro Glu Leu Val Lys Pro Gly Ala Ser
                   85                  90                  95
   Val Lys Met Ser Cys Lys Ala Ser Gly Tyr Thr Phe Thr Asp Tyr Val
               100                 105                 110
   Ile Asn Trp Val Lys Gln Arg Thr Gly Gln Gly Leu Glu Trp Ile Gly
           115                 120                 125
   Glu Ile Tyr Pro Gly Ser Gly Ser Asp Tyr Tyr Asn Glu Asn Leu Lys
       130                 135                 140
   Asp Lys Ala Thr Leu Thr Ala Asp Lys Ser Ser Asn Thr Ala Tyr Met
   145                 150                 155                 160
   Gln Leu Ser Ser Leu Thr Ser Glu Asp Ser Ala Val Tyr Phe Cys Ala
                   165                 170                 175
   Arg Lys Gly Glu Asn Gly Asn Ser Leu Ala Phe Ala Tyr Trp Gly Gln
               180                 185                 190
   Gly Thr Leu Val Thr Val Ser Ala Gly Gly Gly Gly Ser Gly Gly Gly
           195                 200                 205
   Gly Ser Gly Gly Gly Gly Asp Ile Val Leu Thr Gln Ser Pro Ala Ser
       210                 215                 220
   Leu Ala Val Ser Leu Gly Gln Arg Ala Thr Ile Ser Cys Lys Ala Ser
   225                 230                 235                 240
   Gln Ser Val Asp Tyr Asp Gly Asp Ser Tyr Met Asn Trp Tyr Gln Gln
                   245                 250                 255
   Lys Ser Gly Gln Pro Pro Lys Leu Leu Ile Tyr Ala Ala Ser Asn Leu
               260                 265                 270
   Glu Ser Gly Ile Pro Ala Arg Phe Ser Gly Ser Gly Ser Gly Thr Asp
               275                 280                 285
   Phe Thr Leu Asn Ile His Pro Val Glu Glu Glu Asp Ala Ala Thr Tyr
       290                 295                 300
   Tyr Cys Gln Gln Ser Ile Gln Asp Pro Cys Thr Phe Gly Gly Gly Thr
   305                 310                 315                 320
   Glu Leu Glu Ile Lys His His His His His His
                   325                 330
<210> 19
   <211> 1017
   <212> DNA
   <213> Artificial Sequence
<220>
   <223> MR construct
<400> 19
   gactacaaag atgatgacga taaggatatc gtgctgaccc agtctccagc ttctttggct     60
   gtgtctctag ggcagagggc caccatctcc tgcaaggcca gccaaagtgt tgattatgat    120
   ggtgatagtt atatgaactg gtaccaacag aaatcaggac agccacccaa actcctcatc    180
   tatgctgcat ccaatctaga atctgggatc ccagccaggt ttagtggcag tgggtctggg    240
   acagacttca ccctcaacat ccatcctgtg gaggaggagg atgctgcgac ctattactgt    300
   cagcaaagta ttcaggatcc gtgcacgttc ggagggggga ccgagctcga gatcaaaggt     360
   ggtggtggtt ctggcggcgg cggctccggt ggtggtggtt ctcaggttca gctgcagcag    420
   tctggacctg agctggtgaa gcctggggct tcagtgaaga tgtcctgcaa ggcttctgga    480
   tacacattca ctgactatgt tataaattgg gtgaagcaga gaactggaca gggccttgag    540
   tggattggag agatttatcc tggaagtggt agtgattact ataatgagaa cctcaaggac    600
   aaggccacac tgactgcaga caaatcctcc aacacagcct acatgcagct cagtagcctg    660
   acatctgagg actctgcggt ctatttctgt gcaagaaagg gggagaatgg taactccctg    720
   gcgtttgctt actggggcca agggaccctg gtcaccgtct ccgcaggtgg tggtggttct    780
   ggcggcggcg gctcctcccc atattcctcg gacaccacac cctgctgctt tgcctacatt    840
   gcccgcccac tgccccgtgc ccacatcaag gagtatttct acaccagtgg caagtgctcc    900
   aacccagcag tcgtctttgt cacccgaaag aaccgccaag tgtgtgccaa cccagagaag    960
   aaatgggttc gggagtacat caactctttg cagatgagcc atcaccatca ccatcac      1017
<210> 20
   <211> -339
   <212> PRT
   <213> Artificial Sequence
<220>
   <223> MR construct
<400> 20
<210> 21
   <211> 198
   <212> DNA
   <213> Artificial Sequence
<220>
   <223> RANTES del2 nucleotide sequence
<400> 21
   tattcctcgg acaccacacc ctgctgcttt gcctacattg cccgcccact gccccgtgcc      60
   cacatcaagg agtatttcta caccagtggc aagtgctcca acccagcagt cgtctttgtc     120
   acccgaaaga accgccaagt gtgtgccaac ccagagaaga aatgggttcg ggagtacatc     180
   aactctttgg agatgagc                                                   198
<210> 22
   <211> 66
   <212> PRT
   <213> Artificial Sequence
<220>
   <223> RANTES del2
<400> 22
   Tyr Ser Ser Asp Thr Thr Pro Cys Cys Phe Ala Tyr Ile Ala Arg Pro
   1               5                   10                  15
   Leu Pro Arg Ala His Ile Lys Glu Tyr Phe Tyr Thr Ser Gly Lys Cys
               20                  25                  30
   Ser Asn Pro Ala Val Val Phe Val Thr Arg Lys Asn Arg Gln Val Cys
           35                  40                  45
   Ala Asn Pro Glu Lys Lys Trp Val Arg Glu Tyr Ile Asn Ser Leu Glu
       50                  55                  60
   Met Ser
   65
<210> 23
   <211> 198
   <212> DNA
   <213> Artificial Sequence
<220>
   <223> RANTES del2 Glu66Gln
<400> 23
   tattcctcgg acaccacacc ctgctgcttt gcctacattg cccgcccact gccccgtgcc     60
   cacatcaagg agtatttcta caccagtggc aagtgctcca acccagcagt cgtctttgtc    120
   acccgaaaga accgccaagt gtgtgccaac ccagagaaga aatgggttcg ggagtacatc    180
   aactctttgc agatgagc                                                  198
<210> 24
   <211> 66
   <212> PRT
   <213> Artificial Sequence
<220>
   <223> RANTES del2 Glu66Gln
<400> 24
   Tyr Ser Ser Asp Thr Thr Pro Cys Cys Phe Ala Tyr Ile Ala Arg Pro
   1               5                   10                  15
   Leu Pro Arg Ala His Ile Lys Glu Tyr Phe Tyr Thr Ser Gly Lys Cys
               20                  25                  30
   Ser Asn Pro Ala Val Val Phe Val Thr Arg Lys Asn Arg Gln Val Cys
           35                  40                  45
   Ala Asn Pro Glu Lys Lys Trp Val Arg Glu Tyr Ile Asn Ser Leu Gln
       50                  55                  60
   Met Ser
   65
<210> 25
   <211> 360
   <212> DNA
   <213> Mus musculus
<400> 25
   atccagctgc agcagtctgg acctgagctg gtgaagcctg gggcttcagt gaagatgtcc      60
   tgcaaggctt ctggatacac attcactgac tatgttataa attgggtgaa gcagagaact     120
   ggacagggcc ttgagtggat tggagagatt tatcctggaa gtggtagtga ttactataat     180
   gagaacctca aggacaaggc cacactgact gcagacaaat cctccaacac agcctacatg     240
   cagctcagta gcctgacatc tgaggactct gcggtctatt tctgtgcaag aaagggggag     300
   aatggtaact ccctggcgtt tgcttactgg ggccaaggga ccctggtcac cgtctccgca     360
<210> 26
   <211> 120
   <212> PRT
   <213> Mus musculus
<400> 26
   Ile Gln Leu Gln Gln Ser Gly Pro Glu Leu Val Lys Pro Gly Ala Ser
   1               5                   10                  15
   Val Lys Met Ser Cys Lys Ala Ser Gly Tyr Thr Phe Thr Asp Tyr Val
               20                  25                  30
   Ile Asn Trp Val Lys Gln Arg Thr Gly Gln Gly Leu Glu Trp Ile Gly
           35                  40                  45
   Glu Ile Tyr Pro Gly Ser Gly Ser Asp Tyr Tyr Asn Glu Asn Leu Lys
       50                  55                  60
   Asp Lys Ala Thr Leu Thr Ala Asp Lys Ser Ser Asn Thr Ala Tyr Met
   65                  70                  75                  80
   Gln Leu Ser Ser Leu Thr Ser Glu Asp Ser Ala Val Tyr Phe Cys Ala
                   85                  90                  95
   Arg Lys Gly Glu Asn Gly Asn Ser Leu Ala Phe Ala Tyr Trp Gly Gln
               100                 105                 110
   Gly Thr Leu Val Thr Val Ser Ala
           115                 120
<210> 27
   <211> 315
   <212> DNA
   <213> Artificial Sequence
<220>
   <223> nucleotide sequence of modified (VL) of MT413
<400> 27
   tctccagctt ctttggctgt gtctctaggg cagagggcca ccatctcctg caaggccagc 60
   caaagtgttg attatgatgg tgatagttat atgaactggt accaacagaa atcaggacag 120
   ccacccaaac tcctcatcta tgctgcatcc aatctagaat ctgggatccc agccaggttt 180
   agtggcagtg ggtctgggac agacttcacc ctcaacatcc atcctgtgga ggaggaggat 240
   gctgcgacct attactgtca gcaaagtatt caggatccgt gcacgttcgg aggggggacc 300
   gagctcgaga tcaaa 315
<210> 28
   <211> 105
   <212> PRT
   <213> Artificial Sequence
<220>
   <223> Modified (VL) of MT413
<400> 28
   Ser Pro Ala Ser Leu Ala Val Ser Leu Gly Gln Arg Ala Thr Ile Ser
   1               5                   10                  15
   Cys Lys Ala Ser Gln Ser Val Asp Tyr Asp Gly Asp Ser Tyr Met Asn
               20                  25                  30
   Trp Tyr Gln Gln Lys Ser Gly Gln Pro Pro Lys Leu Leu Ile Tyr Ala
           35                  40                  45
   Ala Ser Asn Leu Glu Ser Gly Ile Pro Ala Arg Phe Ser Gly Ser Gly
       50                  55                  60
   Ser Gly Thr Asp Phe Thr Leu Asn Ile His Pro Val Glu Glu Glu Asp
   65                  70                  75                  80
   Ala Ala Thr Tyr Tyr Cys Gln Gln Ser Ile Gln Asp Pro Cys Thr Phe
                   85                  90                  95
   Gly Gly Gly Thr Glu Leu Glu Ile Lys
               100                 105
<210> 29
   <211> 993
   <212> DNA
   <213> Artificial Sequence
<220>
   <223> chemokine construct
<400> 29
   gatatcgtgc tgacccagtc tccagcttct ttggctgtgt ctctagggca gagggccacc      60
   atctcctgca aggccagcca aagtgttgat tatgatggtg atagttatat gaactggtac     120
   caacagaaat caggacagcc acccaaactc ctcatctatg ctgcatccaa tctagaatct     180
   gggatcccag ccaggtttag tggcagtggg tctgggacag acttcaccct caacatccat     240
   cctgtggagg aggaggatgc tgcgacctat tactgtcagc aaagtattca ggatccgtgc     300
   acgttcggag gggggaccga gctcgagatc aaaggtggtg gtggttctgg cggcggcggc     360
   tccggtggtg gtggttctca ggttcagctg cagcagtctg gacctgagct ggtgaagcct     420
   ggggcttcag tgaagatgtc ctgcaaggct tctggataca cattcactga ctatgttata     480
   aattgggtga agcagagaac tggacagggc cttgagtgga ttggagagat ttatcctgga     540
   agtggtagtg attactataa tgagaacctc aaggacaagg ccacactgac tgcagacaaa     600
   tcctccaaca cagcctacat gcagctcagt agcctgacat ctgaggactc tgcggtctat     660
   ttctgtgcaa gaaaggggga gaatggtaac tccctggcgt ttgcttactg gggccaaggg     720
   accctggtca ccgtctccgc aggtggtggt ggttctggcg gcggcggctc ctccccatat     780
   tcctcggaca ccacaccctg ctgctttgcc tacattgccc gcccactgcc ccgtgcccac     840
   atcaaggagt atttctacac cagtggcaag tgctccaacc cagcagtcgt ctttgtcacc     900
   cgaaagaacc gccaagtgtg tgccaaccca gagaagaaat gggttcggga gtacatcaac     960
   tctttgcaga tgagccatca ccatcaccat cac                                  993
<210> 30
   <211> 331
   <212> PRT
   <213> Artificial Sequence
<220>
   <223> Chemokine construct
<400> 30
   Asp Ile Val Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Met Asn Trp Tyr Gln Gln Lys Ser Gly Gln Pro Pro
           35                  40                  45
   Lys Leu Leu Ile Tyr Ala Ala Ser Asn Leu Glu Ser Gly Ile Pro Ala
       50                  55                  60
   Arg Phe Ser Gly Ser Gly Ser Gly Thr Asp Phe Thr Leu Asn Ile His
   65                  70                  75                  80
   Pro Val Glu Glu Glu Asp Ala Ala Thr Tyr Tyr Cys Gln Gln Ser Ile
                   85                  90                  95
   Gln Asp Pro Cys Thr Phe Gly Gly Gly Thr Glu Leu Glu Ile Lys Gly
               100                 105                 110
   Gly Gly Gly Ser Gly Gly Gly Gly Ser Gly Gly Gly Gly Ser Gln Val
           115                 120                 125
   Gln Leu Gln Gln Ser Gly Pro Glu Leu Val Lys Pro Gly Ala Ser Val
       130                 135                 140
   Lys Met Ser Cys Lys Ala Ser Gly Tyr Thr Phe Thr Asp Tyr Val Ile
   145                 150                 155                 160
   Asn Trp Val Lys Gln Arg Thr Gly Gln Gly Leu Glu Trp Ile Gly Glu
                   165                 170                 175
   Ile Tyr Pro Gly Ser Gly Ser Asp Tyr Tyr Asn Glu Asn Leu Lys Asp
               180                 185                 190
   Lys Ala Thr Leu Thr Ala Asp Lys Ser Ser Asn Thr Ala Tyr Met Gln
                   195                 200                 205
   Leu Ser Ser Leu Thr Ser Glu Asp Ser Ala Val Tyr Phe Cys Ala Arg
       210                 215                 220
   Lys Gly Glu Asn Gly Asn Ser Leu Ala Phe Ala Tyr Trp Gly Gln Gly
   225                 230                 235                 240
   Thr Leu Val Thr Val Ser Ala Gly Gly Gly Gly Ser Gly Gly Gly Gly
                   245                 250                 255
   Ser Ser Pro Tyr Ser Ser Asp Thr Thr Pro Cys Cys Phe Ala Tyr Ile
               260                 265                 270
   Ala Arg Pro Leu Pro Arg Ala His Ile Lys Glu Tyr Phe Tyr Thr Ser
           275                 280                 285
   Gly Lys Cys Ser Asn Pro Ala Val Val Phe Val Thr Arg Lys Asn Arg
       290                 295                 300
   Gln Val Cys Ala Asn Pro Glu Lys Lys Trp Val Arg Glu Tyr Ile Asn
   305                 310                 315                 320
   Ser Leu Gln Met Ser His His His His His His
                   325                 330
<210> 31
   <211> 28
   <212> DNA
   <213> Artificial Sequence
<220>
   <223> Primer 1
<220>
   <221> misc_feature
   <222> (7)..(7)
   <223> n is g or a
<220>
   <221> misc_feature
   <222> (11)..(11)
   <223> n is c or g
<220>
   <221> misc_feature
   <222> (17)..(17)
   <223> n is g or t
<220>
   <221> misc_feature
   <222> (20)..(20)
   <223> n is a or c
<220>
   <221> misc_feature
   <222> (23)..(23)
   <223> n is g or c
<400> 31
   ccatggnatg nagctgngtn atnctctt 28
<210> 32
   <211> 37
   <212> DNA
   <213> Artificial Sequence
<220>
   <223> Primer 2
<400> 32
   acctgaggag acggtgaccg tggtcccttg gccccag 37
<210> 33
   <211> 425
   <212> DNA
   <213> Artificial Sequence
<220>
   <223> VH domain of MT-413 in the PCR-Script vector
<400> 33
   ccatgggatg gagctgggtc atgctcttca tcctgtcagg aactgcaggt gtccactccc 60
   aggttcagct gcagcagtct ggacctgagc tggtgaagcc tggggcttca gtgaagatgt 120
   cctgcaaggc ttctggatac acattcactg actatgttat aaattgggtg aagcagagaa 180
   ctggacaggg ccttgagtgg attggagaga tttatcctgg aagtggtagt gattactata 240
   atgagaacct caaggacaag gccacactga ctgcagacaa atcctccaac acagcctaca 300
   tgcagctcag tagcctgaca tctgaggact ctgcggtcta tttctgtgca agaaaggggg 360
   agaatggtaa ctccctggcg tttgcttact ggggccaagg gaccacggtc accgtctcct 420
   caggt 425
<210> 34
   <211> 24
   <212> DNA
   <213> Artificial Sequence
<220>
   <223> Primer 3
<400> 34
   gacattcagc tgacccagtc tcca 24
<210> 35
   <211> 22
   <212> DNA
   <213> Artificial Sequence
<220>
   <223> Primer 4
<400> 35
   gttttatttc cagcttggtc cc 22
<210> 36
   <211> 334
   <212> DNA
   <213> Artificial Sequence
<220>
   <223> VL domain of MT-413 in the PCR-Script vector
<400> 36
   gacattcagc tgacccagtc tccagcttct ttggctgtgt ctctagggca gagggccacc 60
   atctcctgca aggccagcca aagtgttgat tatgatggtg atagttatat gaactggtac 120
   caacagaaat caggacagcc acccaaactc ctcatctatg ctgcatccaa tctagaatct 180
   gggatcccag ccaggtttag tggcagtggg tctgggacag acttcaccct caacatccat 240
   cctgtggagg aggaggatgc tgcgacctat tactgtcagc aaagtattca ggatccgtgc 300
   acgttcggag gggggaccaa gctggaaata aaac 334
<210> 37
   <211> 29
   <212> DNA
   <213> Artificial Sequence
<220>
   <223> Primer 5
<400> 37
   aaggatatcg tgctgaccca gtctccagc 29
<210> 38
   <211> 56
   <212> DNA
   <213> Artificial Sequence
<220>
   <223> Primer 6
<400> 38
   ggagccgccg ccgccagaac caccaccacc tttgatctcg agctcggtcc cccctc 56
<210> 39
   <211> 50
   <212> DNA
   <213> Artificial Sequence
<220>
   <223> Primer 7
<400> 39
   ggcggcggcg gctccggtgg tggtggttct caggttcagc tgcagcagtc 50
<210> 40
   <211> 59
   <212> DNA
   <213> Artificial Sequence
<220>
   <223> Primer 8
<400> 40
   cctgtcgact aatgatgatg gtgatgatgt gcggagacgg tgaccagggt cccttggcc 59
<210> 41
   <211> 786
   <212> DNA
   <213> Artificial Sequence
<220>
   <223> Sequence 3
<400> 41
   gactacaaag atgatgacga taaggatatc gtgctgaccc agtctccagc ttctttggct 60
   gtgtctctag ggcagagggc caccatctcc tgcaaggcca gccaaagtgt tgattatgat 120
   ggtgatagtt atatgaactg gtaccaacag aaatcaggac agccacccaa actcctcatc 180
   tatgctgcat ccaatctaga atctgggatc ccagccaggt ttagtggcag tgggtctggg 240
   acagacttca ccctcaacat ccatcctgtg gaggaggagg atgctgcgac ctattactgt 300
   cagcaaagta ttcaggatcc gtgcacgttc ggagggggga ccgagctcga gatcaaaggt 360
   ggtggtggtt ctggcggcgg cggctccggt ggtggtggtt ctcaggttca gctgcagcag 420
   tctggacctg agctggtgaa gcctggggct tcagtgaaga tgtcctgcaa ggcttctgga 480
   tacacattca ctgactatgt tataaattgg gtgaagcaga gaactggaca gggccttgag 540
   tggattggag agatttatcc tggaagtggt agtgattact ataatgagaa cctcaaggac 600
   aaggccacac tgactgcaga caaatcctcc aacacagcct acatgcagct cagtagcctg 660
   acatctgagg actctgcggt ctatttctgt gcaagaaagg gggagaatgg taactccctg 720
   gcgtttgctt actggggcca agggaccctg gtcaccgtct ccgcacatca tcaccatcat 780
   cattag 786
<210> 42
   <211> 49
   <212> DNA
   <213> Artificial Sequence
<220>
   <223> Primer 9
<400> 42
   ggagccgccg ccgccagaac caccaccacc tgcggagacg gtgaccagg 49
<210> 43
   <211> 35
   <212> DNA
   <213> Artificial Sequence
<220>
   <223> Primer 10
<400> 43
   ggcggcggcg gctcctcccc atattcctcg gacac 35
<210> 44
   <211> 51
   <212> DNA
   <213> Artificial Sequence
<220>
   <223> Primer 11
<400> 44
   gccgtcgact agtgatggtg atggtgatgg ctcatctgca aagagttgat g 51
<210> 45
   <211> 28
   <212> DNA
   <213> Artificial Sequence
<220>
   <223> Primer 12
<400> 45
   aaagatatcc agctgcagca gtctggac 28
<210> 46
   <211> 50
   <212> DNA
   <213> Artificial Sequence
<220>
   <223> Primer 13
<400> 46
   ggcggcggcg gctccggtgg tggtggtgac atcgtgctga cccagtctcc 50
<210> 47
   <211> 49
   <212> DNA
   <213> Artificial Sequence
<220>
   <223> Primer 14
<400> 47
   cccgtcgact aatgatggtg atgatgatgt ttgatctcga gctcggtcc 49
<210> 48
   <211> 30
   <212> DNA
   <213> Artificial Sequence
<220>
   <223> Primer 15
<400> 48
   aaatgcgcag gcctccccat attcctcgga 30
<210> 49
   <211> 46
   <212> DNA
   <213> Artificial Sequence
<220>
   <223> Primer 16
<400> 49
   tcgtccggag ccacctccac ctgagctcat ctgcaaagag ttgatg 46
<210> 50
   <211> 41
   <212> DNA
   <213> Artificial Sequence
<220>
   <223> Primer 17
<400> 50
   aaatccggag gtggtggatc cgatatccag ctgcagcagt c 41
<210> 51
   <211> 5
   <212> PRT
   <213> Artificial Sequence
<220>
   <223> Linker
<400> 51
   Gly Gly Gly Gly Ser
   1 5
<210> 52
   <211> 14
   <212> PRT
   <213> Artificial Sequence
<220>
   <223> Linker
<400> 52
   Gly Gly Gly Gly Ser Gly Gly Gly Gly Ser Gly Gly Gly Gly
   1 5 10
<210> 53
   <212> PRT
   <213> Artificial Sequence
<220>
   <223> Linker
<400> 53
<210> 54
   <211> 15
   <212> PRT
   <213> Artificial Sequence
<220>
   <223> Linker
<400> 54
   Gly Gly Gly Gly Ser Gly Gly Gly Gly Ser Gly Gly Gly Gly Ser
   1 5 10 15